# EUROPEAN PATENT SPECIFICATION

(11) **EP 3 371 603 B1**
(45) Date of publication and mention of the grant of the patent: **11.03.2020**
(21) Application number: 16794273.9
(22) Date of filing: 07.11.2016
(51) Int. Cl.: G01N 33/68

(54) **METHOD FOR CHARACTERIZATION OF CELL SPECIFIC MICROVESICLES**
VERFAHREN ZUR CHARAKTERISIERUNG VON ZELLSPEZIFISCHEN MIKROVESIKELN
PROCÉDÉ DE CARACTÉRISATION DES MICROVÉSICULES SPÉCIFIQUES DE CELLULE

(30) Priority: 06.11.2015 EP 15193500
(43) Date of publication of application: 12.09.2018
(73) Proprietor: Humanitas Mirasole S.p.A., 20100 Rozzano (MI) (IT)
(72) Inventor: CONDORELLI, Gianluigi, 20089 Rozzano (MI) (IT); ANSELMO, Achille, 20089 Rozzano (MI) (IT); VIVIANI ANSELMI, Chiara, 20089 Rozzano (MI) (IT); PAPA, Laura, 20089 Rozzano (MI) (IT)
(74) Representative: Capasso, Olga
(86) International application number: PCT/EP2016/076810
(87) International publication number: WO 2017/077105

(56) References cited:
- WO-A1-2012/024543
- WO-A1-2012/115885
- WO-A2-2013/022995
- WO-A2-2013/134786

## Description

### Field of the invention

The present invention relates to a method for characterizing, quantifying and sorting the circulating cell specific microvesicles (MV), in particular the cardiac-derived MV.

The present invention also refers to methods for diagnosing and/or assessing the risk of developing and/or for prognosing and/or for monitoring the progression and/or for monitoring the efficacy of a therapeutic treatment and/or for the screening of a therapeutic treatment of a disease, in particular of cardiovascular diseases, by characterizing, quantifying and sorting the circulating cardiac-derived microvesicles (MV).

### Background art

Cardiovascular diseases continue to be a leading cause of morbidity and mortality among adults in all economically advanced Countries. The identification of clear biomarkers related to a high risk of cardiovascular diseases remains still largely elusive due to the heterogeneity of the pathological processes underlying these diseases. Therefore, the building of a novel technological approach able to improve the early detection of cardiovascular diseases (CVD) might lead to more accurate interventions and treatment of patients.

Extracellular vesicles (EV) are the key players of the intercellular communications and are released under basal or stress conditions.

EV include apoptotic bodies (ABs), microvesicles (MV) and exosomes, originating from different subcellular compartments [Raposo, et al. J Cell Biol. 2013 Feb 18;200(4):373-83]. In detail, MV are released from their cell of origin upon fusion with the plasma membrane. Noteworthy, the characterization of circulating MV could improve the pathophysiological information for pre-symptomatic disease, leading to a better assessment of patient risk stratification and following therapeutic approaches.

The absence of a method for selectively characterizing and quantifying circulating cardiac-derived MV is a limiting factor for applying this technology to disease staging and prediction of acute events.

Therefore, it is still felt the need of a method for selectively characterize and quantify cardiac-derived MV.

WO 2012/024543, WO 2012/115885, WO 2013/022995 and WO 2013/134786 disclose reagents for detection of markers disclosed herein.

### Summary of the invention

The present inventors showed that under stress conditions, circulating cell specific microvesicles (MV), in particular the cardiac-derived MV levels, are a reliable index of the pathological state of the cardiovascular system.

Indeed, the present inventors found a dramatic drop of circulating cardiac- and endothelium-derived MV concentrations after a minimally invasive procedure called Transcatheter Aortic Valve Implantation (TAVI). This "proof of principle" is of pathophysiological interest.

The present inventors developed a simple, accurate and powerful tool for characterizing, quantifying and sorting the circulating tissue-derived MV, focusing on cardiac-derived MVs, for disease prediction, staging and drug-responsiveness.

The inventors based their method on a sequential stepwise fashion of gating strategy using the multicolor Flow Cytometry (FACS). Inventors optimized the FACS analysis to simultaneously characterize, quantify and sorting the circulating tissue-derived MV, including ABs and/or membrane fragments.

The method of the invention may be used for staging of patients with heart failure, cardiomyopathy and valvular diseases and prediction of disease severity in patients with acute coronary artery syndromes, including the drug-responsiveness profile with a prognostic/predictive role on the major adverse cardiac outcome.

Noteworthy, the present method can be set up not only in the strict meaning of CVD, but also in other pathologies as autoimmune diseases (rheumatoid arthritis, arthritis psoriasis, polymyalgia rheumatic, lupus, scleroderma autoimmune disorders, ..).

Cardiovascular complications are the leading negative outcome of autoimmune diseases and could be due to inflammatory status or drug-responsiveness.

Additionally, a wide variety of cancer therapies (i.e. breast cancer, Hodgkin lymphoma, ..) induce the cardiotoxicity, possibly leading to heart failure and other cardiovascular negative consequences.

The method of the invention is highly adaptable to different aspects closely-linked to CVD directly (i.e. heart failure) and/or indirectly (i.e. individual drug-response), with main implications in clinical management and personalized medicine.

The present method allows to accurately discriminate circulating cardiac-derived MV, using the anti CD172a antibody, the linking of which to myocardial-derived MV was not previously shown. Conventional biomarkers (as Troponin T (cTnT)) of myocardial injury or heart failure (as NT-proBNP) are mainly used in clinical practice, however they still show a reduced performance in a number of medical pathological conditions.

Commercial magnetic-bead coated with antibody capture, customized dried antibody cocktails for multicolor analysis and/or columns with sized filter cartridges and/or combined with specific antibody filter (SAF) could be applied and developed ad hoc for carrying out the present method.

The present method may be used also in research field for MV sorting. The purified MV subsets can be used for the analysis of their content and function (i.e. proteomics analysis, Next-Generation Sequencing, ..). The obtained findings will improve the knowledge of CVD pathogenesis and drug-responsiveness.

All the antibodies used in the panel designed by the present inventors recognize specific markers expressed on the surface of the MV thus drastically decreasing the background signal typical of the intracellular staining.

The designed matrix 7x7 of the antibodies (Table 1) enables to accurately discriminate the cardiac (CD172a+) circulating tissue-derived MV subsets of interest, strategically placed as last parameter. However, the present matrix is highly adaptable to apply to different study designs and different combinations of antibodies selecting in the last two positions (R13 and R14) circulating tissue-derived MV subsets of major interest and can be designed using different numbers of antibodies ("n x n" matrix), relevant to further clinical applications.

It is in fact possible to substitute one or more antibodies (or add or remove one or more antibodies) of the matrix to characterize and measure the amount of microvesicles released by different organs not only by heart. It is known that organs subjected to stress conditions, e.g. kidney, liver etc, show changes in microvesicle release [Wang Y, et al. Stem Cell Res Ther. 2015 22;6:100][Lemoinne S, at al. Nature Reviews Gastroenterology & Hepatology, 2014 11,350-361][Ettelaie C,at al Microvasc Res 2008; 76: 152-60][Royo F, J Extracell Vesicles. 2012 Jul 11;1]

### Detailed description of the invention

It is therefore an object of the invention an ex-vivo or in vitro method for characterizing and/or measuring the amount of subsets of circulating tissue-derived microvesicles (MV) comprising the step of detecting at least the CD172a marker on said microvescicles, in an isolated biological sample obtained from the subject, wherein if the microvescicle is positive for CD172a the microvesicle is characterized as a cardiac derived microvescicle.

The method of the invention is an ex-vivo or in vitro, more preferably ex-vivo, method.

The method of the invention preferably further comprises the step of further detecting at least one marker selected from the group consisting of: CD235a, CD61, CD144, CD14, CD45, CD73, CD3 or any combination thereof.

In a more preferred embodiment of the invention, the markers to be detected are CD172a, CD235a, CD61, CD144, CD14, CD45 and CD73.

The markers may be detected in any order, preferably in the following sequence: CD235a, CD61, CD144, CD14, CD45, CD172a and CD73.

More preferably the markers CD45 and CD14 are detected at the same time.

In a preferred embodiment of the invention, additionally:
- if the microvesicle is positive for CD235a the microvesicle is characterized as an erythroid derived MV;
- if the microvesicle is positive for CD61 the microvesicle is characterized as a platelet derived MV;
- if the microvesicle is positive for CD144 the microvesicle is characterized as an endothelium-derived MV;
- if the microvesicle is positive for CD14 the microvesicle is characterized as a monocyte-derived MV;
- if the microvesicle is positive for CD45 the microvesicle is characterized as a leukocyte derived MV;
- if the microvesicle is positive for CD73 the microvesicle is characterized as a stromal/adipocyte derived MV.

In a preferred embodiment, if the microvesicle is positive for CD3 and/or CD45 the microvesicle is a leukocyte-derived MV.

CD3 marker can be used combined with CD45 to better discriminate leukocyte-derived MVs. A further object of the invention is an ex vivo or in vitro method for diagnosing and/or assessing the risk of developing and/or prognosing and/or for monitoring the progression and/or for monitoring the efficacy of a therapeutic treatment and/or for the screening of a therapeutic treatment of cardiovascular diseases (CVD), in a subject comprising the steps of:
a) characterizing and/or measuring the amount of subsets of circulating tissue-derived microvesicles according to the above defined method;
b) comparing with respect to a proper control and/or reference.

The above method is an ex-vivo or in vitro method, more preferably an ex-vivo method.

In preferred embodiment, an amount of cardiac-derived MV, in the isolated biological sample obtained from the subject, higher than the control amount indicates that the subject is either affected by or is at increased risk for developing cardiovascular diseases (CVD), preferably heart failure, primary or secondary.

In an alternative embodiment of the method of the invention, an amount of cardiac-derived MV, in the isolated biological sample obtained from the subject, lower than the control amount indicates that the subject is going toward an amelioration of the CVD.

Said subject preferably undergone a valve replacement, preferably a percutaneous aortic valve replacement (TAVR) (also defined as "transcatheter aortic valve implantation" (TAVI)).

In a preferred embodiment of the methods of the invention, the measured or characterized subset of circulating tissue-derived microvesicles is the cardiac-derived MV subset.

Said cardiac-derived MV subset is preferably characterized by the presence of the marker CD172a.

In an alternative method of the invention, cardiac and/or endothelium- and/or stromal-, and/or leukocyte- and/or platelet- and/or monocyte- and/or erythroid -derived MV subset may be characterized by the presence of different markers (or detected with different antibodies) compared to the markers (or antibodies) used in the present method.

Said cardiac-derived microvesicles are preferably negative for at least one of the following markers: CD235a, CD61, CD144, CD14, CD45, CD73, CD3. More preferably said cardiac-derived microvesicles are negative for the following markers: CD235a, CD61, CD144, CD14, CD45, CD73.

The cardiovascular disease (CVD) is preferably selected from the group consisting of: heart failure, preferably primary or secondary, aortic stenosis (AS), valvular disease, cardiomyopathy, acute coronary artery disease (CAD), atherosclerosis, myocardial ischemia, infarction, arrhythmias, hypertrophic cardiomyopathy (HCM) and drug-dependent cardiotoxicity connected to different pathologies (e.g. cancer, HIV-HAART therapies).

The isolated biological sample of the above defined methods is preferably plasma, blood, serum, tissue obtained by surgical resection, tissue obtained by biopsy, cells culture, cell supernatants, cell lysates, tissue samples, organs, bone marrow.

In the methods according to the invention, the marker is preferably detected by means of a specific ligand, preferably said ligand is an antibody, or functional fragments thereof.

Said marker is preferably detected by magnetic beads coated with antibody capture (e.g. MACS® MicroBeads, Miltenyi) and/or customized dried antibody cocktails (e.g. BD Lyotubes) and/or columns with sized filter cartridges and/or combined with specific antibody filter (SAF). In the methods according to the invention, the subsets of circulating tissue-derived microvesicles are preferably characterized and/or their amount measured by means of multicolor flow cytometry technology (FACS), immunogold electron microscopy, immunofluorescence, ELISA, immunoprecipitation, reverse colorimetric immunoassay (RCIA), radioimmune assay (RIA) Electrochemiluminescence, surface plasmon resonance (SPR)-based approach, nanoliter microfluidics (immunoassays) or spectometry.

A cell-of-origin specific microvesicle can be enriched or isolated using one or more binding agents using a magnetic capture method, fluorescence activated cell sorting (FACS) or laser cytometry as described above. Magnetic capture methods can include, but are not limited to, the use of magnetically activated cell sorter (MACS) microbeads or magnetic columns.

Any other appropriate method for isolating or otherwise enriching the cell-of-origin specific microvesicles with respect to a biological sample may also be used in combination with the present invention. For example, size exclusion chromatography such as gel permeation columns, centrifugation or density gradient centrifugation, and filtration methods can be used in combination with the antigen selection methods described herein.

Accordingly, microvesicles can be isolated from cells derived from a site of cardiovascular disease. In some embodiments, the isolated microvesicles are derived from cells related to such diseases and disorders, whose analysis is informative for a diagnosis, prognosis, disease stratification, theranosis, prediction of responder / non-responder status, disease monitoring, treatment monitoring and the like as relates to such diseases and disorders. The microvesicles are further useful to discover biomarkers.

Techniques useful for validation and characterization of a microvesicle includes, but is not limited to, western blot, electron microscopy, immunohistochemistry, immunoelectron microscopy, FACS (Fluorescent activated cell sorting), electrophoresis (1 dimension, 2 dimension), liquid chromatography, mass spectrometry, MALDI-TOF (matrix assisted laser desorption/ionization-time of flight), ELISA, LC-MS-MS, and nESI (nanoelectrospray ionization).

A further object of the invention is the use of a ligand specific for CD172a for the detection and/or quantification of cardiac-derived MV in an isolated biological sample. Said ligand is preferably an anti-CD 172a antibody, or functional fragments thereof.

Also herein described is a kit comprising detecting means for the markers as above defined, preferably for carrying out the methods as above defined.

Preferably, the CD45 and/or CD14 markers are detected before the detection of the CD172a marker.

The sequence information of the marker CD172a is recorded in a NCBI-PUBMED database (Gene ID: 140885; Protein ID: NP_001035111.1).

The sequence information of the marker CD235a is recorded in a NCBI-PUBMED database (Gene ID: 2993; Protein ID: NP_002090.4).

The sequence information of the marker CD61 is recorded in a NCBI-PUBMED database (Gene ID: 3690; Protein ID: NP_000203.2).

The sequence information of the marker CD144 is recorded in a NCBI-PUBMED database (Gene ID: 1003; Protein ID: NP_001786.2).

The sequence information of the marker CD14 is recorded in a NCBI-PUBMED database (Gene ID: 929; Protein ID: NP_000582.1).

The sequence information of the marker CD45 is recorded in a NCBI-PUBMED database (Gene ID: 5788; Protein ID: NP_001254727.1).

The sequence information of the marker CD73 is recorded in a NCBI-PUBMED database (Gene ID: 4907; Protein ID: NP_001191742.1).

The sequence information of the marker CD3 is recorded in a NCBI-PUBMED database (Gene ID: 915/916 and 917; Protein ID: NP_000723.1/NP_000724.1 and NP_000064.1).

It is comprised in the methods of the invention the substitution of one or more of the above markers (or the addition or removal of one or more markers). For example, it is possible to modify the present method in order to characterize and measure the amount of microvesicles released by different organs not only by heart, or it is possible to substitute one or more markers with other known markers.

In the same way, it is comprised in the methods of the invention the substitution of one or more of the presently used antibodies (or the addition or removal of one or more antibodies) in order to detect the same described microvesicles or other microvesicles.

The methods of the invention may also be used for identifying the severity of the disease and/or stratifying patients.

The methods of the invention may also be used for sorting the circulating tissue-derived microvesicles (MV).

Microvesicles are circular membrane fragments, ranging in size from 100nm to 1µm in diameter of different subcellular origin released by most known eukaryotic cell types.

In the context of the present invention, the term "extracellular vesicles" comprises apoptotic bodies (ABs), microvesicles (MV) and exosomes.

In the context of the present invention the expression "subsets of circulating tissue-derived microvesicles" refers to different tissue-derived MV. In particular, the subsets are characterized by presenting at least one of the above markers and/or other know markers.

In the context of the present invention, the microvesicle subsets are e.g. erythroid derived MV, platelet derived MV, endothelium-derived MV, monocyte-derived MV, leukocyte derived MV, cardiac derived MV, or stromal/adipocyte derived MV.

A preferred embodiment of the present methods, comprises comparing the amount of each quantified marker to a predetermined cutoff for said marker and determining whether said subject has a risk or is affected, or determining the stage, or determining the prognosis or monitoring the efficacy of a therapeutic treatment, of cardiovascular disease based on the comparison.

Preferably, a cut-off of cardiac derived MV ≥4.7 (absolute count/mL) was selected to optimize and identify a potential "grey zone" of cardiac biomarker in patients affected by aortic stenosis. Preferably, a cut-off of cardiac derived CD172a+ MV ≥1.79/ml (absolute count/mL) was selected to accurately discriminate patients which are good responder to TAVI treatment (thus presenting a strong reduction of mortality after treatment) from patients with CD172a+ MV <1.79/ml (absolute count/mL) as "no-responder" (higher percentage of mortality).

A "severity index" of cardiac-stress may be identified by the skilled man, in particular by applying the present method and cardiac-biomarker on an independent cohort of patients with heart failure and stratified according New York Heart Association (NYHA) class or other classifications.

In the present invention, the control value or proper control may also be selected from a value measured in a healthy patient, a patient affected by a non-CVD, a patient affected by a CVD before a therapeutic treatment, a patient affected by a CVD during the time course of a therapeutic treatment, a patient affected by a CVD at various time point during the course of the disease.

The microvesicles are obtained from any convenient biological sample. Plasma and serum samples from an individual are preferred samples, which may be treated in various ways, including binding to affinity reagents for identification and sorting. For example, samples may be stained with antibodies that selectively bind to the markers.

The sample, e.g. plasma sample, may be subjected to MV isolation with all the methods known to the expert of the art.

A microvesicle can be isolated from an archived or stored sample. Alternatively, a microvesicle may be isolated from a biological sample and analyzed without storing or archiving of the sample.

An enriched population of microvesicles can be obtained from a biological sample. For example, microvesicles may be concentrated or isolated from a biological sample using size exclusion chromatography, density gradient centrifugation, differential centrifugation, nanomembrane ultrafiltration, immunoabsorbent capture, affinity purification, micro fluidic separation, or combinations thereof.

Size exclusion chromatography, such as gel permeation columns, centrifugation or density gradient centrifugation, and filtration methods can be used. For example, a microvesicle can be isolated by differential centrifugation, anion exchange and/or gel permeation chromatography (for example, as described in US Patent Nos. 6,899,863 and 6,812,023), sucrose density gradients, organelle electrophoresis (for example, as described in U.S. Patent No. 7,198,923), magnetic activated cell sorting (MACS), or with a nanomembrane ultrafiltration concentrator. Various combinations of isolation or concentration methods can be used.

The microvesicles may also be sorted and analyzed for the presence of circulating biomarkers (as cardiac troponin T (cTnT)), proteins, lipids or nucleic acids of interest, such as RNA, including microRNA, as miR-1 and miR-133a.

Thus, a treatment can be selected for the subject suffering from a CVD, based on the signature identified by the methods of the invention. Accordingly, the signature can comprise a presence or level of a circulating biomarker, including a microRNA, a vesicle, or any useful microvesicle associated biomarker.

In the present disclosure any combination (e.g. of two, three, four,...) of the above defined markers (or antibodies) may be detected or their amount or alteration measured. Said combinations are e.g. CD172a and CD235a and CD61, or CD172a and CD61 and CD144,... The markers may be detected in any sequence.

In the case of a method or a kit for diagnosing and/or assessing the risk of developing and/or prognosing a disease, the proper control may be a sample taken from a healthy patient or from a patient affected by another disorder or pathology, and the control amount may be the amount of the same marker or subset of MV measured in a sample taken from a healthy patient or from a patient affected by another disorder or pathology.

In the case of a method or a kit for monitoring the progression of the disease, the progress of the disease is monitored and the proper control may be a sample taken from the same subject at various times or from another patient, and the control amount may by the amount of the same marker or subset of MV measured in a sample taken from the same subject at various times or from another patient.

If by comparing the measured amount of cardiac-derived MV with the amount obtained from a control sample, the amount of said subset of MV in the sample isolated from the subject corresponds to an higher value, the subject may present CVD, may be at risk of developing CVD or go towards an aggravation of said disease.

In the case of a method or a kit for monitoring the efficacy of a therapeutic treatment, the proper control may by a sample taken from the same subject before initiation of the therapy or taken at various times during the course of the therapy and the control amount may be the amount of the same marker or MV subset measured in a sample taken from the same subject before initiation of the therapy or taken at various times during the course of the therapy.

In this case, if the amount of cardiac-derived MV, in the isolated biological sample obtained from the subject is lower than the control amount, it may indicate that the therapeutic treatment is effective. The treatment may be a valve replacement such as TAVI.

In the case of a method or a kit for the screening of a therapeutic treatment, the proper control may be a sample taken from subjects without treatment, or from subjects treated with a substance that is to be assayed or from subjects treated with a reference treatment and the proper control amount may be the average of the amounts of the same marker or cardiac-derived MV, measured in samples taken from subjects without treatment, or from subjects treated with a substance that is to be assayed or from subjects treated with a reference treatment. In this case, if the amount of cardiac-derived MV, in the isolated biological sample obtained from the subject is lower than the control amount, it may indicate that the tested substance is effective for the treatment of the CVD.

In the context of the present invention, the term "characterizing" may be intended also as "detecting" and the term "detecting" may be intended also as "measuring the amount" or "measuring the alteration".

Aspects of the invention include analysis of the quantity and/or quality (for example the presence of protein or nucleic acid markers of interest) of microvesicles for monitoring cardiac function and therapeutic response in the clinical settings of acute and chronic heart failure. Aspects of the invention include analysis of the quantity and/or quality (for example the presence of protein or nucleic acid markers of interest) of microvesicles for monitoring of cardiotoxicity responses to drugs, e.g. to cancer therapies (e.g. chemotherapy and radiation therapy). Aspects of the invention include analysis of the quantity and/or quality (for example the presence of protein or nucleic acid markers of interest) of microvesicles for monitoring cardiovascular complications due to autoimmune diseases, inflammatory status, including analysis of the quality and/or quantity for prediction of disease in patients with heart failure and monitoring therapeutic responsiveness.

Aspects of the invention include analysis of the quantity and/or quality (for example the presence of protein or nucleic acid markers of interest) of microvesicles for aortic stenosis following percutaneous aortic valve replacement (TAVI) and other invasive valve replacement procedures and heart failure.

Aspects of the invention include analysis of the quantity and/or quality (for example the presence of protein or nucleic acid markers of interest) of microvesicles for prediction of disease severity in patients with acute coronary artery syndrome or for monitor drug-responsiveness profile.

Such analysis may include detecting the number of microvesicles relative to total serum protein levels, and may include determining the presence of CD172a on the microvesicles.

Aspects of the invention include analysis of the quantity and/or quality (for example the presence of protein or nucleic acid markers of interest) of microvesicles which is incorporated into a point of care device for purposes of identifying individuals with radiation exposure or specific infections.

In some embodiments, a patient sample, e.g. a plasma or serum sample, is analyzed for the presence of microvesicles, which may be exosomes, comprising markers of interest. Analysis may include mass spectroscopy, but preferably utilizes flow cytometry with the methods of the invention. Markers of interest include CD172a, CD235a, CD61, CD144, CD14, CD45, CD73 or CD3 and any combination thereof.

Assessment in a patient allows improved care, where patients classified according to responsiveness can be treated with an appropriate agent. Patients can be classified upon initial presentation of symptoms, and can be further monitored for status over the course of the disease to maintain appropriate therapy, or can be classified at any appropriate stage of disease progression.

Treatment of particular interest includes pharmacological for heart failure due to primary cardiomyopathy or secondary to various diseases processes, and other invasive replacement procedures including those for cardiac valves, such as TAVI.

In other examples disclosed herein a device or kit is provided for the analysis of patient samples. Alternatively the reagents can be provided as a kit comprising reagents in a suspension or suspendable form, e.g. reagents bound to beads suitable for flow cytometry, preferably magnetic beads coated with antibody capture, or customized dried antibody cocktails for Multicolor analysis and/or columns with sized filter cartridges, and/or combined with specific antibody filter (SAF) and the like. The instructions may comprise instructions for conducting an antibody-based flow cytometry assay.

Detecting means are preferably means able to detect and/or measure the amount of the described markers, e.g. means able to detect the complex antigen-antibody, as enzyme conjugated secondary antibodies, luminescent substrates, magnetic beads coated with antibody capture, customized dried antibody cocktails and/or columns with size filter cartridges and/or combined with specific antibody filter (SAF).

In an embodiment, the method further comprises selecting a therapeutic regimen based on the analysis. In an embodiment, the method further comprises determining a treatment course for the subject based on the analysis.

Marker signature pattern as used herein refers to the spectrum of marker on microvesicles. Once the marker levels and pattern for a particular sample are identified, the data can be used in selecting the most appropriate therapy for an individual. By analysis of marker levels on an individual basis, the specific subclass of disease is determined, and the patient can be classified based on the likelihood to respond to treatments of interest. Thus, the marker signature can provide prognostic information to guide clinical decision making, both in terms of institution of and escalation of therapy as well as in the selection of the therapeutic agent to which the patient is most likely to exhibit a robust response.

The information obtained from the marker profile is used to (a) determine type and level of therapeutic intervention warranted (i.e. more versus less aggressive therapy, monotherapy versus combination therapy, type of combination therapy), and (b) to optimize the selection of therapeutic agents. With this approach, therapeutic regimens can be individualized and tailored according to the specificity data obtained at different times over the course of treatment, thereby providing a regimen that is individually appropriate. In addition, patient samples can be obtained at any point during the treatment process for analysis.

The term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids, polypeptides, etc, or for microvescicles. The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like.

The term "diagnosis" is used herein to refer to the identification of a molecular or pathological state, disease or condition. The term also includes staging and/or predicting disease severity and/or predicting outcome of the disease.

The term "prognosis" is used herein to refer to the prediction of the likelihood of disease-attributable death or progression, including recurrence and drug resistance of the disease. The term "prediction" is used herein to refer to the act of foretelling or estimating, based on observation, experience, or scientific reasoning. In one example, a physician may predict the likelihood that a patient will survive, following a therapeutic treatment or surgery.

The terms "biomarker," "biomarkers," "marker" or "markers" refer to, without limitation, to the mentioned proteins (and sequence information above mentioned), and to peptides, nucleic acids, oligonucleotides thereof together with their related metabolites, mutations, variants, orthologues, polymorphisms, modifications, fragments, subunits, isoforms, precursors, degradation products, elements, and other analytes or sample-derived measures. Markers can also include mutated proteins, mutated nucleic acids, variations in copy numbers and/or transcript variants. Markers also encompass non-blood borne factors and non-analyte physiological markers of health status, and/or other factors or markers not measured from samples (e.g., biological samples such as bodily fluids), such as clinical parameters and traditional factors for clinical assessments. Markers can also include any indices that are calculated and/or created mathematically. Markers can also include combinations of any one or more of the foregoing measurements, including temporal trends and differences.

To "analyze" includes determining a set of values associated with a sample by measurement of a marker (such as, e.g., presence or absence of a marker or constituent expression levels) in the sample and comparing the measurement against measurement in a sample or set of samples from the same subject or other control subject(s). The markers of the present teachings can be analyzed by any of various conventional methods known in the art. To "analyze" can include performing a statistical analysis to, e.g., determine whether a subject is a responder or a non-responder to a therapy.

A "sample" in the context of the present teachings refers to any biological sample that is isolated from a subject. A sample can include, without limitation an aliquot of body fluid, whole blood, serum, plasma, tissue biopsies, synovial fluid, lymphatic fluid, ascites fluid, and interstitial or extracellular fluid. The term "sample" also encompasses the fluid in spaces between cells, including gingival crevicular fluid, bone marrow, cerebrospinal fluid (CSF), saliva, mucous, sputum, semen, sweat, urine, or any other bodily fluids. "Blood sample" can refer to whole blood or any fraction thereof, including serum and plasma. Samples can be obtained from a subject by means including but not limited to venipuncture, excretion, ejaculation, massage, biopsy, needle aspirate, lavage, scraping, surgical incision, or intervention or other means known in the art.

A "ligand" as described herein can also be linked directly or indirectly to a solid surface or substrate. For example, an antibody used to isolate a microvesicle can be bound to a solid substrate such as a well, such as commercially available plates (e.g. from Nunc, Milan Italy). Each well can be coated with the antibody. In some embodiments, the antibody used to isolate a microvesicle is bound to a solid substrate such as an array. The array can have a predetermined spatial arrangement of molecule interactions, binding islands, biomolecules, zones, domains or spatial arrangements of binding islands or binding agents deposited within discrete boundaries. Further, the term array may be used herein to refer to multiple arrays arranged on a surface, such as would be the case where a surface bore multiple copies of an array. Such surfaces bearing multiple arrays may also be referred to as multiple arrays or repeating arrays.

The ligand can also be bound to particles such as beads or microspheres. For example, an antibody specific for a component of a microvesicle can be bound to a particle, and the antibody-bound particle is used to isolate a microvesicle from a biological sample. In some embodiments, the microspheres may be magnetic or fluorescently labeled. In addition, a binding agent for isolating microvesicles can be a solid substrate itself. For example, latex beads, such as aldehyde/sulfate beads (Interfacial Dynamics, Portland, OR) can be used.

A ligand bound to a magnetic bead can also be used to isolate a microvesicle.

A ligand, such as an antibody, for isolating microvesicles is preferably contacted with the biological sample comprising the microvesicles of interest for at least a time sufficient for the ligand to bind to a component of the microvesicle. For example, an antibody may be contacted with a biological sample for various intervals ranging from seconds days, including but not limited to, about 10 minutes, 30 minutes, 1 hour, 3 hours, 5 hours, 7 hours, 10 hours, 15 hours, 1 day, 3 days, 7 days or 10 days.

A ligand such as an antibody specific to the above markers can be labeled with, including but not limited to, a magnetic label, a fluorescent moiety, an enzyme, a chemiluminescent probe, a metal particle, a non-metal colloidal particle, a polymeric dye particle, a pigment molecule, a pigment particle, an electrochemically active species, semiconductor nanocrystal or other nanoparticles including quantum dots or gold particles. The label can be, but not be limited to, fluorophores, quantum dots, or radioactive labels. For example, the label can be a radioisotope (radionuclides), such as <3>H,<11>C,<14>C,<18>F,<32>P,<35>S,<64>Cu,<68>Ga,<86>Y,<99>Tc,<111>In,<123>I, <124>I,<125>I,<131>I,<133>Xe,<177>Lu,<211>At, or<213>Bi. The label can be a fluorescent label, such as a rare earth chelate (europium chelate), fluorescein type, such as, but not limited to, FITC, 5-carboxyfluorescein, 6-carboxy fluorescein; a rhodamine type, such as, but not limited to, TAMRA; dansyl; Lissamine; cyanines; phycoerythrins; Texas Red; and analogs thereof. The fluorescent label can be one or more of FAM, dRHO, 5-FAM, 6FAM, dR6G, JOE, HEX, VIC, TET, dTAMRA, TAMRA, NED, dROX, PET, BHQ, Gold540 and LIZ.

The ligand can be directly or indirectly labeled, e.g., the label is attached to the antibody through biotin-streptavidin. Alternatively, an antibody is not labeled, but is later contacted with a second antibody that is labeled after the first antibody is bound to an antigen of interest. Antibodies include, but are not limited to, polyclonal, monoclonal, bispecific, synthetic, humanized and chimeric antibodies, single chain antibodies, The term "antibody" also comprise also functional fragments which are able to bind their antigens, as well as molecules comprising such antigen-binding fragments, including engineered antibody fragments, antibody derivatives, bispecific antibodies and other multispecific molecules, scFv, Fv fragment, a Fab fragment, a F(ab)2 fragment, a multimeric antibody, a peptide or a proteolytic fragment containing the epitope binding region.

An antibody, or generally any molecule, "binds specifically" to an antigen (or other molecule) if the antibody binds preferentially to the antigen, and, e.g., has less than about 30%, 20%, 10%, 5% or 1% cross-reactivity with another molecule. The ligand can also be a polypeptide or peptide.

In a preferred embodiment of the methods of the invention, the antibody used is at least one antibody selected from the group consisting of:
anti-CD235a (GA-R2) (BD Biosciences); [Van Beers EJ, et al. Haematologica.2009 Nov;94(11):1513-9];
anti-CD61 (VI-PL2) (BD Biosciences); [Crompot E, et al. PLoS One. 2015 May 15; 10(5):e0127209];
anti-CD144 (REA199) (Miltenyi Biotech); [Koga H, et al. J Am Coll Cardiol. 2005 May 17;45(10):1622-30];
anti-CD45 (HI30) (BD Biosciences) and/or anti-CD 14 (M5E2) (BD Biosciences); [Tähtinen S, et al. Cancer Immunol Res. 2015 May 14] [Griffin JD,et al J. Clin. Invest. 1981; 68: 932-41]; anti-CD73 (AD2) (BD Biosciences); [Müller G, et al.. Obesity (Silver Spring). 2011 Aug;19(8):1531-44];
anti-CD172a (15-414) (eBioscience), [Dubois NC, et al. Nat Biotechnol. 2011 Oct 23;29(11):1011-8],
or any combination thereof.

Isolation or detection of a microvesicle using a particle such as a bead or microsphere can also be performed using flow cytometry. Flow cytometry can be used for sorting microscopic particles suspended in a stream of fluid. As particles pass through they can be selectively charged and on their exit can be deflected into separate paths of flow. It is therefore possible to separate populations from an original mix, such as a biological sample, with a high degree of accuracy and speed. Flow cytometry allows simultaneous multiparametric analysis of the physical and/or chemical characteristics of single cells flowing through an optical/electronic detection apparatus. A beam of light, usually laser light, of a single frequency (color) is directed onto a hydrodynamically focused stream of fluid. A number of detectors are aimed at the point where the stream passes through the light beam; one in line with the light beam (Forward Scatter or FSC) and several perpendicular to it (Side Scatter or SSC) and one or more fluorescent detectors.

"Detecting" or "detection", "measuring" or "measurement" in the context of the present teachings refers to determining the presence, absence, quantity, amount, or effective amount of a substance in a clinical or subject-derived sample, including the presence, absence, or concentration levels of such substances, and/or evaluating the values or categorization of a subject's clinical parameters based on a control.

Classification can be made according to predictive modeling methods that set a threshold for determining the probability that a sample belongs to a given class. The probability preferably is at least 50%, or at least 60% or at least 70% or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class. The predictive ability of a model can be evaluated according to its ability to provide a quality metric, e.g. AUC or accuracy, of a particular value, or range of values. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC (area under the curve) of at least about 0.7, at least about 0.75, at least about 0.8, at least about 0.85, at least about 0.9, or higher. As is known in the art, the relative sensitivity and specificity of a predictive model can be "tuned" to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about at least about 0.7, at least about 0.75, at least about 0.8, at least about 0.85, at least about 0.9, or higher. Unless otherwise apparent from the context, all elements, steps or features of the invention can be used in any combination with other elements, steps or features.

A sample from an individual is analyzed for the presence of microvesicles, which are optionally detectable labeled for one or more markers of interest. Parameters of interest include microvesicle size, quantity, presence of RNA of interest, presence of proteins of interest, presence of lipids of interest.

When MV are characterized and counted by FACS, a 0.22 to 0.45µm filtered sheath fluid can be used to limit background noise from dust and crystals.

In order to gating MV, beads, e.g. Megamix Plus FSC beads, or propylene non fluorescent different size beads, e.g. NIST Traceable Polystyrene-Melamine Size Standards Nanosphere 0.05µm-1µm, are preferably used.

Preferably, in the methods of the invention, before detecting the above defined markers, ABs and/or membrane fragments are detected.

In the present method, dyes detecting nucleus could be used to detect compromised plasma membranes, e.g. SYTOX, Phallotoxin, Propidium, amino reactive dyes, 7-Aminoactinomycin D etc.

As internal quality check, the inventors investigated the expression of phosphatidylserine on the surface of all the MV-subsets analysed using saturating concentration of Annexin-V. [Heijnen HF, et al Blood. 1999 Dec 1;94(11):3791-9.]

In a preferred embodiment of the invention, only events SYTOX/Phallotoxin double negatives were analyzed for the detection of tissue-derived MVs. Preferably appropriate saturating concentrations of highly specific surface antibodies from 15 to 45 minutes at 4°C or RT were used.

The flow cytometry technology is able to detect the particles size and the granularity by FSC (Forware SCatter) and SSC (Side Scatter) parameters. FSC is a measurement of the amount of the laser beam that passes around the cell. This gives a relative size for the particles.

The SSC parameter is a measurement of the amount of the laser beam that bounces off of particulates inside of the particles.

Using beads of known size one can determine the size of a population based on the FSC parameters.

In a preferred aspect of the invention, membrane fragments while detected, are discarded from the MV analyzed population.

The signature pattern can be generated from a biological sample using any convenient protocol. The readout can be a mean, average, median or the variance or other statistically or mathematically-derived value associated with the measurement. The marker readout information can be further refined by direct comparison with the corresponding reference or control pattern. A binding pattern can be evaluated on a number of points: to determine if there is a statistically significant change at any point in the data matrix; whether the change is an increase or decrease in the binding; whether the change is specific for one or more physiological states, and the like. The absolute values obtained for each marker under identical conditions will display a variability that is inherent in live biological systems and also reflects the variability inherent between individuals.

Following obtainment of the signature pattern from the sample being assayed, the signature pattern is compared with a reference or control profile to make a prognosis regarding the phenotype of the patient from which the sample was obtained/derived. Typically a comparison is made with a sample or set of samples from an unaffected, normal source or the follow up value is considered the own reference value of each patient. Additionally, a reference or control signature pattern can be a signature pattern that is obtained from a sample of a patient known to be responsive or non-responsive to the therapy of interest, and therefore can be a positive reference or control profile.

In certain embodiments, the obtained signature pattern is compared to a single reference/control profile to obtain information regarding the phenotype of the patient being assayed. In yet other embodiments, the obtained signature pattern is compared to two or more different reference/control profiles to obtain more in depth information regarding the phenotype of the patient. For example, the obtained signature pattern can be compared to a positive and negative reference profile to obtain confirmed information regarding whether the patient has the phenotype of interest.

The detection reagents can be provided as part of a kit. Thus, the disclosure further provides kits for detecting the presence of a panel of specific markers of interest in a biological sample. Procedures using these kits can be performed by clinical laboratories, experimental laboratories, medical practitioners, or private individuals. The kits for detecting markers comprise affinity reagents useful for generating a prognostic signature pattern, which can be provided in solution or bound to a substrate. The kit can optionally provide additional components that are useful in the procedure, including, but not limited to, buffers, developing reagents, labels, reacting surfaces, means for detection, control samples, standards, instructions, and interpretive information.

Patient outcomes and status can be assessed using imaging-based criteria such as radiographic scores, clinical and laboratory criteria. Multiple different imaging, clinical and laboratory criteria and scoring systems have been and are being developed to assess disease activity and response to therapy in CVD, inflammatory diseases, etc.

A pattern can be obtained as a dataset for an indication of interest. The dataset comprises quantitative data for the presence in serum or other biological sample of at least 1 microvesicle marker, etc. A statistical test will provide a confidence level for a change in the expression, titers or concentration of markers between the test and control profiles to be considered significant, where the control profile can be for selected as appropriate. The raw data can be initially analyzed by measuring the values for each marker, usually in duplicate, triplicate, quadruplicate or in 5-10 replicate features per marker.

A test dataset is considered to be different than a control dataset if one or more of the parameter values of the profile exceed the limits that correspond to a predefined level of significance.

In the context of the present invention, the term "sensitivity" means the identifying true positives when biomarker levels are used clinically to identify disease/outcome/condition of interest.

In the context of the present invention, the term "specificity" means the identifying true-negatives when biomarker levels are used clinically to identify disease/outcome/condition of interest.

In the context of the present invention, the term "AUC" or "accuracy" means the better tradeoff between false positive and true positive rates to measure predictive accuracy when biomarker levels are used clinically to identify disease/outcome/condition of interest. AUC is determined from ROC curve, a plot of the sensitivity versus (1-specificity) of a biomarker. Additionally, "Accuracy" in the "antibodies matrix" of the present invention means the cross-reactivity and/or co-expression between antibodies inversely linked with Accuracy of the system.

In the context of the present invention, the term "IC95%" ("95% confidence intervals") means an estimated range of values that covers 95% of the normal density curve of a population. On the contrary, the probability of observing a value outside of this area is less than 0.05 (level of significance). Usually all values are estimated at 90% and/or 95% and/or 99% of confidence level, preferable as 95%.

The methods according to the invention are ex-vivo or in vitro methods, more preferably ex-vivo methods.

In the methods of the invention, the subject (or patient) may be an animal or a human, preferably a human.

The invention will be now illustrated by means of non-limiting examples referring to the following figures.

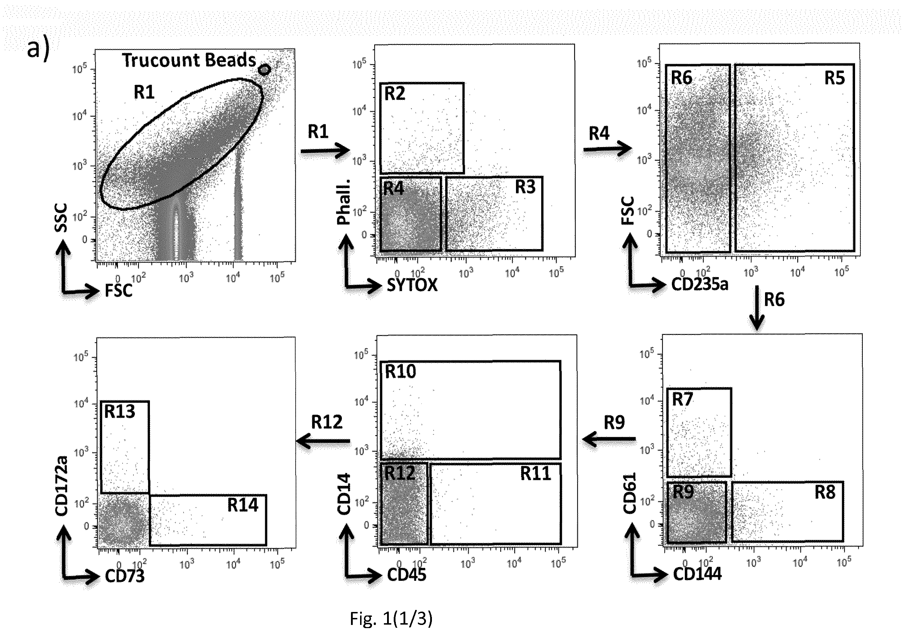

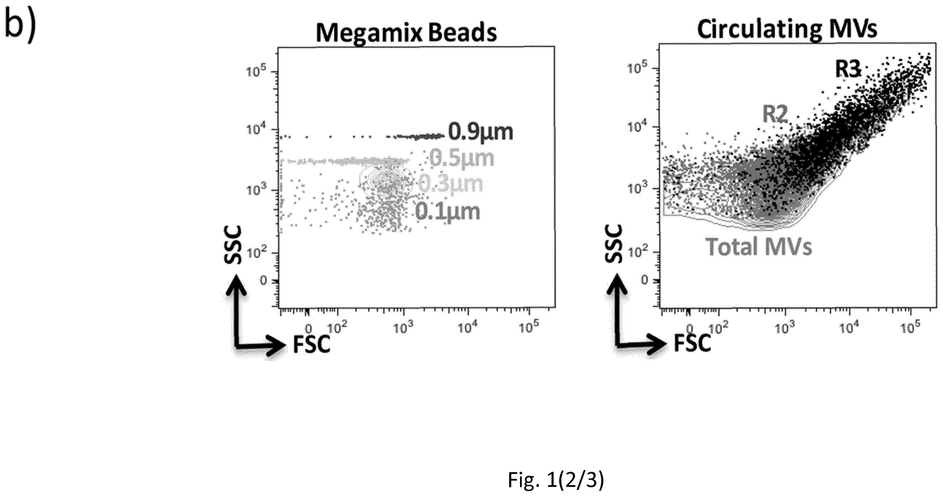

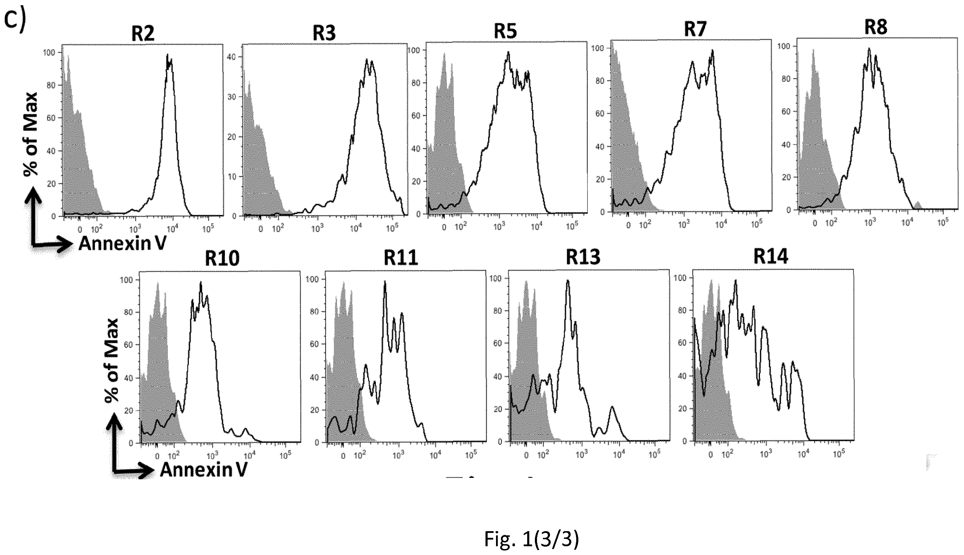

**Figure 1****.** "Stepwise gating strategy" by FACS of circulating tissue-derived MV subsets. (a) FACS analysis of plasma derived MV subpopulations assessed by immunostaining. (b) Difference in vesicle size between SYTOX+ apoptotic bodies (R3), cell membrane fragments or apoptotic bodies Phallotoxin+/SYTOX- (R2) and total microvesicles Phallotoxin-/SYTOX-(R5+R7+R8+R10+R11+R13+R14) was confirmed comparing their morphological distribution with Megamix beads at the same FSC/SSC values. Representative images out of three independent experiments.(c) Analysis of Annexin V expression on circulating tissue-derived MV subsets. Images are representative of four analyzed patients.

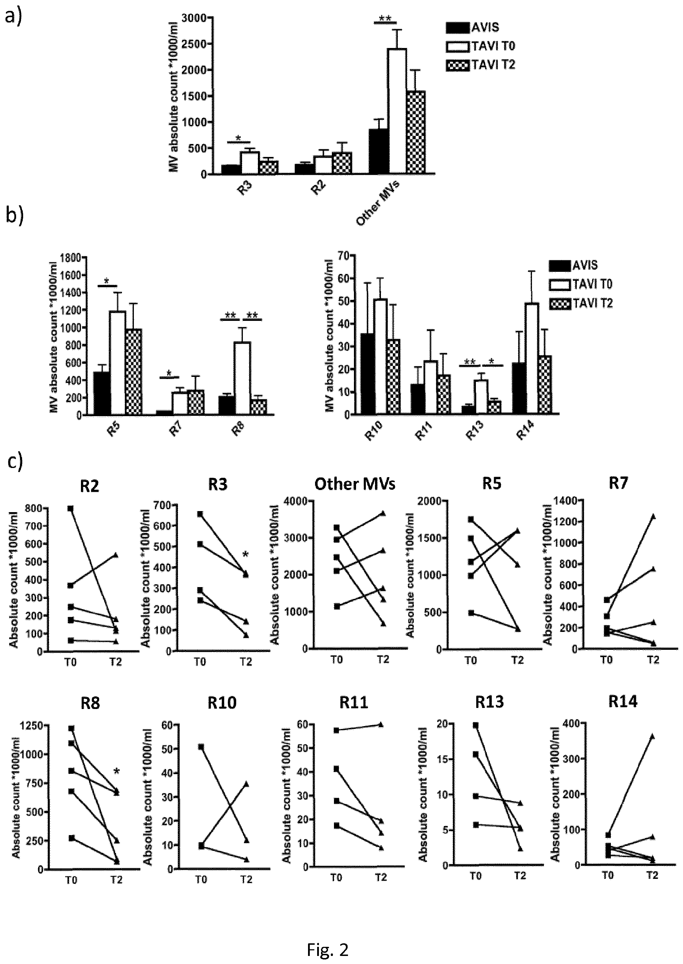

**Figure 2****.** Absolute count of plasma derived microvesicle subpopulations.
   (a-c) Flow cytometric absolute count of SYTOX+ apoptotic bodies (R3), cell membrane fragments or apoptotic bodies Phallotoxin+/SYTOX- (R2) and total microvesicles Phallotoxin-/SYTOX- (R5+R7+R8+R10+R11+R13+R14). (b-c) Flow cytometric absolute count of microvesicle subpopulations R5, R7, R8, R10, R11, R13, and R14. In all panels the MV absolute count was performed using Trucount beads. The analysis were performed on healthy donor (n=4) and TAVI patients before and upon two months of Follow Up (n=3-5). (a-b) Unpaired and (c) paired Wilcoxon test; (*P<0.05; **P<0.01). Error bars represent SEM.

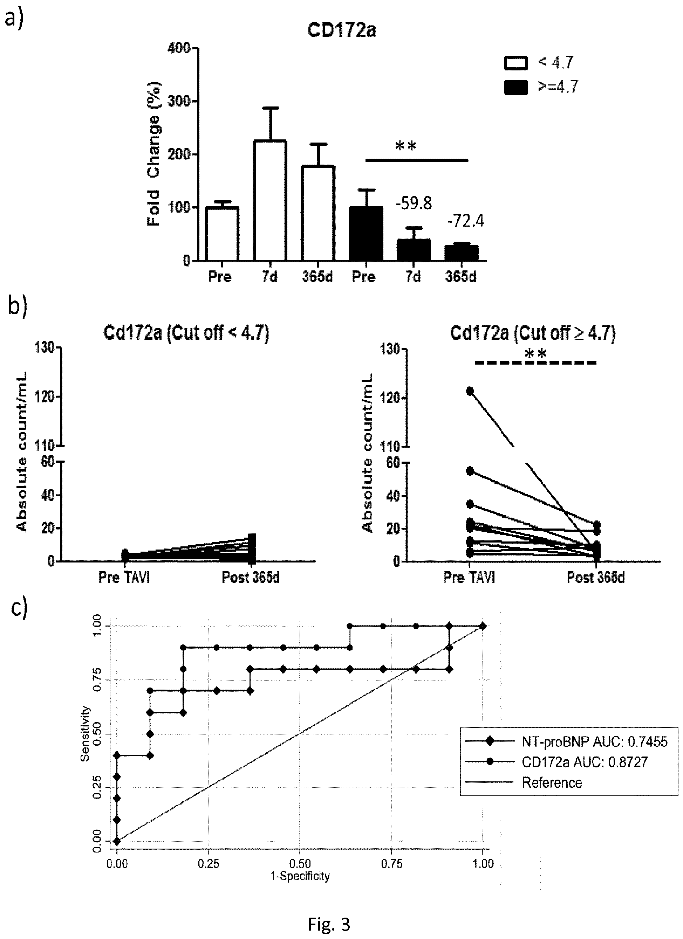

**Figure 3****.** Cardiac-derived microvesicle in AS patients with preselected cut-off.
   (a-b) Flow cytometric absolute count of microvesicle subpopulations R13 in patients with preselected cut-off (CD172a -derived MV absolute count/mL< and ≥4.7). Wilcoxon Matched Pairs analysis was performed on n=10 (≥4.7) and n=11 (<4.7). (*P<0.05; **P<0.001). Error bars represent SEM. (c) R13 ROC area against gold standard (NT-proBNP).

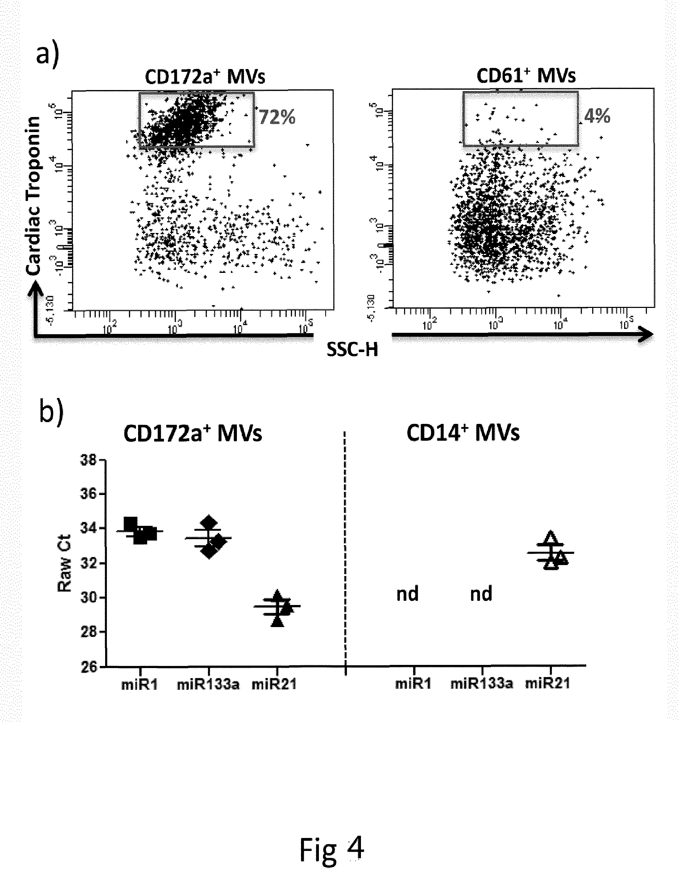

**Figure 4****.** CD172a+ MVs drive cardiac specific molecules.
   (a) Representative intracellular flow cytometry analysis of cardiac troponin T expression on CD172a+microvesicles. Platelet derived microvesicles (CD61+) were used as internal negative control. Representative images out of three independent observations. (b) Real time PCR analysis of miR-1, miR-133a and miR-21 in sorted CD172a+ microvesicles. Sorted monocyte derived microvesicles (CD14+) were used as internal negative controls for the cardiac specific miR-1 and miR-133a(nd= not detectable). Data shown are from three independent observations. Error bars represent SEM.
**Figure 5****.** CD172a+ MVs in healthy donors.
   Flow cytometric analysis of the cardiac derived MVs (CD172a+) obtained from a control group stratified for gender (female (F), male (M)) and age (years (Y) < 65; Y ≥ 65). The MV absolute count was performed using Trucount beads. Error bars represent SEM.
**Figure 6****.** CD172a+ MVs in Aortic stenosis patients.
   Flow cytometric analysis of the cardiac derived MVs obtained from healthy donors (HD) (n= 52) and Aortic Stenosis (AS) patients (n=109). The MV absolute count was performed using Trucount beads. Mann Whitney test; (***P<0.001). Error bars represent SEM.
**Figure 7****.** CD172a+ MVs in Aortic stenosis patients before and upon 1 year of TAVI surgery. (a) Flow cytometric absolute count of cardiac derived microvesicles (CD172a+) (R13) (left panel) and other total MVs (R5+R7+R8+R10+R11+R13) (right panel) in Aortic Stenosis (AS) patients before and upon 1 year of TAVI surgery (n=109). In all panels the MV absolute count was performed using Trucount beads. (b) Kaplan-Meier Survival Curves performed between Groups identified by cut off. Mann-Whitney U-test (*P<0.05; Error bars represent SEM) and Long rank mantel cox test respectively (**P<0.01).
**Figure 8****.** CD172a+ MVs in Aortic stenosis Italian patients before and upon TAVI surgery.
   Flow cytometric absolute count of cardiac derived microvesicles (CD172a+) (R13) (top panel) and other total MVs (R5+R7+R8+R10+R11+R13) (bottom panel) in Aortic Stenosis (AS) patients before and upon 48h and 2 months of TAVI surgery (n=10). In all panels the MV absolute count was performed using Trucount beads. Mann-Whitney U-test; (*P<0.05). Error bars represent SEM.

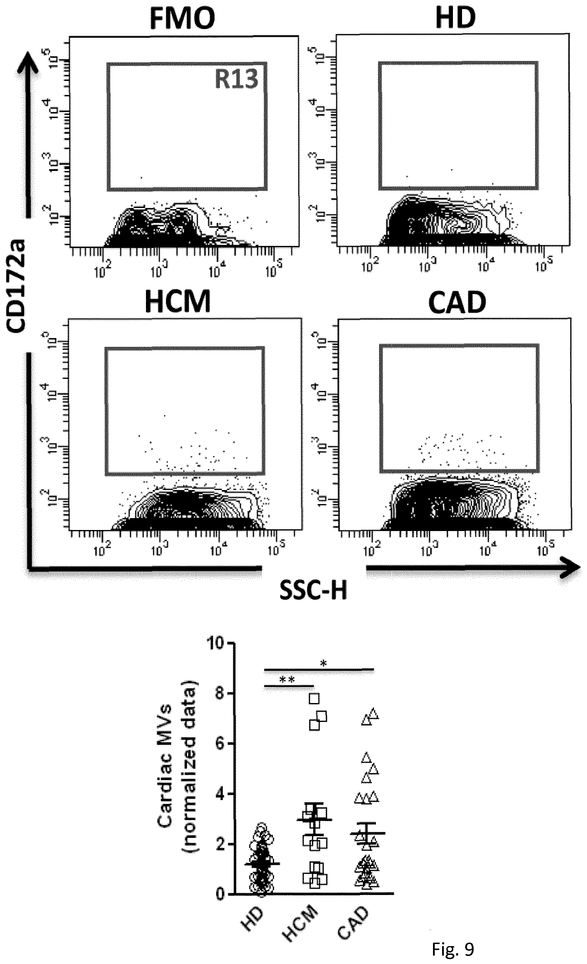

**Figure 9****.** CD172a+ MVs in cardiac pathological conditions
   Flow cytometric absolute count of cardiac derived microvesicles (CD172a+) in Healthy Donors (HD) (n= 52) Hypertrophic Cardiomyopathy (HCM) (n=15) and Coronary Artery Disease (CAD) (n=28) patients. MV absolute count was performed using Trucount beads. In order to verify the selective changes in Cardiac MV absolute counts in each pathological condition analyzed, data were normalized using the following formula: Cardiac MVs (R13)/Total other MVs (R5+R7+R8+R10+R11+R14)^{∗}1000. The multiplier was arbitrarily selected in order to better illustrate the data in decimal range>0. (per 1000). Mann-Whitney U-test; (*P<0.05; **P<0.01). Error bars represent SEM.

### Examples

### EXAMPLE 1

### Materials and Methods

### Plasma Isolation and storage

According to published recommendations, plasma sampling and storage techniques were standardized for overall patients and healthy subjects.

Informed consent was obtained from each patient and healthy subjects.

A 5 ml sample of peripheral blood was collected in EDTA-containing Vacutainer tubes. The vials were processed within 2 h of collection by centrifugation at 1200 x g at room temperature in a bench top centrifuge for 20 minutes to eliminate all blood cells. To further reduce leukocyte and red cells contamination, the top third of the plasma was aspirated and placed in fresh tubes and frozen at -80°C.

### MV isolation from plasma.

Human plasma was diluted with filtered PBS-/- (no calcium and magnesium) and centrifuged at 500g x 30 minutes at 4°C. The obtained Platelet Free Plasma (PFP) was centrifuged at 12000g x 45 minutes at 4°C. Finally, the supernatant was removed leaving 25µl of a MV-enriched suspension which was further diluted with 75µl of filtered PBS-/-. [Caby MP, et al. Int Immunol. 2005 Jul;17(7):879-87]

### Circulating MV characterization and count by FACS

To limit background noise from dust and crystals, a 0.22 µm filtered sheath fluid was indifferently used preserving sterility, for sample acquisition.

A morphological gate of microvesicles was performed using Megamix Plus FSC beads (0.1 µm to 1 µm beads Biocytex), according to literature [Mobarrez F, et al., Thromb Res. 2010 Mar;125:e110-6].

Events included in a range from 0.1µm to 5µm were clearly discriminated from background noise.

Apoptotic bodies and possible cell membrane fragments derived from freezing/thawing procedure were stained using SYTOX (Invitrogen-Molecular Probes) and Phallotoxin (Invitrogen-Molecular Probes) at room temperature (RT) for 15 and 30 minutes, respectively [Mobarrez F, et al., Thromb Res. 2010 Mar;125:e110-6].

In detail, the SYTOX molecule is a high affinity nucleic acid dye while the Phallotoxin binds F-actin. Both dyes easily penetrates only compromised plasma membranes.

According to the present "stepwise gating strategy", only events SYTOX/Phallotoxin double negatives were analyzed for the detection of tissue-derived MV using the appropriate saturating concentrations of highly specific surface antibodies (30 minutes at RT).

### Tissue-derived MV surface Antibodies selection.

The antibodies were selected according to literature, as following:
- R5 Subset: CD235a (GA-R2) (BD Biosciences); [Van Beers EJ, et al. Haematologica.2009 Nov;94(11):1513-9]
- R7 Subset: CD61 (VI-PL2) (BD Biosciences); [Crompot E, et al. PLoS One. 2015 May 15;10(5):e0127209]
- R8 Subset: CD144 (REA199) (Miltenyi Biotech); [Koga H, et al. J Am Coll Cardiol. 2005 May 17;45(10):1622-30]
- R10+R11 Subset: CD45 (HI30) (BD Biosciences) combined with CD14 (M5E2) (BD Biosciences); [Tähtinen S, et al. Cancer Immunol Res. 2015 May 14] [Griffin JD,et al J. Clin. Invest. 1981; 68: 932-41].
- R14 Subset: CD73 (AD2) (BD Biosciences); [Müller G, et al.. Obesity (Silver Spring). 2011 Aug;19(8):1531-44]
- R13 Subset: CD172a (15-414) (eBioscience), not yet published its involvement in circulating cardiac-derived MVs. [Dubois NC, et al. Nat Biotechnol. 2011 Oct 23;29(11):1011-8]

To avoid potential co-expression of CD172a on monocyte- and leukocyte-MVs derived, as previously reported in the literature, the present method enables to discriminate monocyte- and leukocyte-MVs derived (R10 and R11) in the first steps of the matrix.

All gate regions were restrictively defined using both negative controls: Fluorescence Minus One (FMO) and isotype controls.

Internal MV Quality check: Phosphatidylserine (PS), a marker on the extracellular leaflet of MVs, was verified in all characterized MV-subsets with saturating concentration of Annexin V (BD Biosciences) for 15 minutes at RT.

Circulating MV absolute count was performed using Trucount beads (BD Biosciences), following manufacturer's instructions.

### Results

The present inventors' "stepwise gating strategy", reported in Figure 1A, is a potent and strategic tool able to accurately discriminate the uninvestigated cardiac (R13)-derived MV, minimizing the false positive events and using an anti-CD 172a antibody not known as marker for circulating cardiac-derived MV.

The first assessed gate allows the identification of ABs (R3) and/or membrane fragments (R2). The sequential gating strategy is able to exclude the predominant MV erythroid (R5), platelet (R7), endothelium (R8) and leukocyte (R10 and R11) and to specifically focus on cardiac-(R13) and stromal/adipocyte (R14) -derived MV.

As showed in Figure 1b, these three main subpopulation were size compared with Megamix Plus FSC beads and, as expected, R2 and R3 had a dimension > 0.5µm while the other MVs (R5;R7;R8;R10;R11;R13;R14) ranged between 0.1 and 0.5µm.

Moreover the matrix 7x7 of the antibodies, reported in Table 1, is representative of the applied "stepwise gating strategy". Noteworthy, the high specificity of the antibodies with a cross reaction <10% and accuracy of system (100% for cardiac-derived MV) were successfully reached.

Internal MV Quality check: The Annexin V, a surface marker for the microvesicles [Heijnen HF, et al Blood. 1999 Dec 1;94(11):3791-9.], was evaluated in overall characterized MV subsets, as showed in Figure 1c.

### Preliminary Data on CVD patients

### 1) Pilot Study

Plasma samples were collected from patients with aortic stenosis (AS) at the time of inclusion (time=0) from "Federico II" University, Naples. AS patients were then undergone percutaneous aortic valve replacement (TAVI). Clinical follow-up and plasma sampling was performed at a 2-months follow-up (time=2M). Five TAVI patients and four healthy subjects were recruited. Our "stepwise gating strategy" was applied and the absolute count was summarized in Figure 2. Among the MV characterized from plasma, AB (R3), erythroid-(R5), endothelium- (R8) and cardiac- (R13) derived MV subsets were found at significantly higher levels in pre-TAVI AS patients as compared with healthy donors (Fig.2a and 2b). Strikingly, levels of circulating cardiac- and endothelium-derived MV subsets were significantly reduced after TAVI (Fig.2a and 2b).

Noteworthy, the lower levels of AB (R3) and endothelium- (R8) derived MVs were confirmed applying a Wilcoxon Matched Pairs test (Fig.2c) while the cardiac (R13)-derived MV subset was slightly under the significant threshold, probably due to small sample size.

### 2) Improvement of preliminary data in independent AS patients

The same findings were obtained increasing the sample size with an independent group of AS patients enrolled from the University Hospital of Kiel, Germany. The present "stepwise gating strategy" was applied on 21 pre TAVI AS patients followed at 7 days and 365 days post-surgery. Strikingly, levels of circulating cardiac- and endothelium- derived MV subsets were significantly reduced after 365 days of follow up. Significant Wilcoxon Matched Pairs p-values were obtained. Monocitic- and stromal- derived showed the same significant trends after 1 years post-surgery.

Additionally, the significant interlinking between cardiac, endothelium-, monocitic- and stromal-derived MV was observed by Spearman' rho correlation analysis.

In detail, the interlinked MV-MV showed a rho≥0.80 a P<0.0000, as reported in the correlation matrix of Table II.

The identified inter-linking between MV-MV may characterize also other CVDs.

To optimize and identify a potential "grey zone" of cardiac biomarkers (CD172a) identifying for (R13)-derived MV, a "threshold" analysis was performed. A "quintile-based method" and "Time-based strategy".

A similar cut-off was obtained, as following reported:
- Quintile-based method: cut-off ≥4.7 (absolute count/mL), AUC:0.51 IC95%: 0.34-0.65, Std Error 0.07, Sensitivity (Se): 56% and Specificity (Sp): 48%;
   Inventors obtained Se and Sp <0.70 but for biomarkers monitoring disease progression (as well as drug responsiveness) the patient serves as his own control (baseline values versus follow-up values), and the best Se or Sp threshold (>0.7, etc) becomes less important.

Inventors selected a R13 cut-off to accurately identify the potential "Grey Zone" (MV absolute count <4.7/mL) and the best performer cut-off of our cardiac-biomarker (MV absolute count ≥4.7/mL). The threshold of the cardiac- biomarker able to discriminate patients with AS was optimized in the phase of the updated data using a larger cohort of AS patients and age-matched control group.

According to previous data about the interlinking of MV-MV, the "best performer cut-off" of cardiac biomarkers (≥4.7) enables to identify the "best performer cut-off" and the "grey zone" of the other correlated MV (endothelium-, monocitic- and stromal-derived MVs).

Wilcoxon Matched Pairs analysis was performed into two obtained groups (CD172a cut-off < (n=11) and ≥ 4.7 absolute count/mL (n=10), respectively). (Fig.3a-Fig.3b) A strikingly reduction of 72% of CD172a was observed post 365 days.

Finally test equality of ROC area against gold standard (NT-proBNP) was performed in patients with pre-designed CD172a cut-off (≥ 4.7 absolute count/mL). (Fig.3c) Cardiac-biomarkers showed the higher AUC = 0.8727 (Std. Err. 0.08) than gold standard AUC = 0.7455 (Std. Err. 0.12).

### Tables

**Table.1 The matrix 7x7 of "Stepwise gating strategy" to characterize circulating tissue-derived MVs from Human Plasma.**

| % | **CD235a⁺** | **CD61⁺** | **CD144⁺** | **CD14⁺** | **CD45⁺** | **CD172a⁺** | **CD73⁺** |
|---|---|---|---|---|---|---|---|
| **R5** | 100 | 5.5±0.8 | 12.1±4.7 | 2.3±0.8 | 2±0.2 | 2.7±0.7 | 8.6±3.8 |
| **R7** | 0 | 100 | 0 | 1.3±0.3 | 0.3±0.1 | 0.6±0.3 | 1.5±0.4 |
| **R8** | 0 | 0 | 100 | 2.5±0.7 | 2.7±0.6 | 3.3±1.5 | 4±0.8 |
| **R10** | 0 | 0 | 0 | 100 | 3±0.6 | 4.2±1.7 | 1.9±0.8 |
| **R11** | 0 | 0 | 0 | 0 | 100 | 8.7±3.2 | 7±2 |
| **R13** | 0 | 0 | 0 | 0 | 0 | 100 | 0 |
| **R14** | 0 | 0 | 0 | 0 | 0 | 0 | 100 |

Cross reaction or/and co-expression of the antibodies used in the panel were put in a matrix. Data shown are the mean of six samples (± Standard Error).

R5:erythroid-derived; R7: platelet-derived; R8: endothelium-derived; R10: monocyte-derived; R11: leukocyte-derived; R13: cardiac-derived; R14: stromal/adipocyte-derived circulating MVs.

**Table. II Inter linking MV-MV**

| | **CD172a** | **CD144** | **CD73** | **CD14** |
|---|---|---|---|---|
| **CD172a** | 1 | | | |
| **CD144** | **Rho=0.81** P<0.0000 | 1 | | |
| **CD73** | **Rho=0.83** P<0.0000 | **Rho=0.92** P<0.0000 | 1 | |
| **CD14** | **Rho=0.84** P<0.0000 | **Rho=0.94** P<0.0000 | **Rho=0.95** P<0.0000 | 1 |

Data shown are the P and the Spearman's CorrelationCoefficient (r)s of tissue derived-MVs in AS patients.

### EXAMPLE 2

### MV Isolation and storage.

According to published recommendations, plasma samples and storage techniques were standardized. A 5 ml sample of peripheral blood was collected in EDTA-containing Vacutainer tubes. The vials were processed within 2 h of collection by centrifugation at 1,200g x 20 min. at room temperature (RT) to eliminate all blood cells. To further reduce leukocyte and red cell contamination, the top third of the plasma was aspirated and placed in a fresh tube. According to the literature, isolated plasma was subsequently diluted with filtered PBS and centrifuged at 500g x 30 min. at 4 °C. The obtained platelet-free plasma was centrifuged at 12,000g x 45 min. at 4 °C. Finally, the supernatant was removed, leaving 25 µl of an MV-enriched suspension, which was diluted with 75 µl of filtered PBS (Caby MP, et al. Int Immunol. 2005 Jul;17(7):879-87).

### FACS analysis.

To limit background noise from dust and crystals, a 0.22 µm filtered sheath fluid was used for sample acquisition. A morphological gate of microvesicles was performed according to Megamix-Plus FSC beads size (range: 0.1-0.9 µm) (Biocytex), according to literature (Mobarrez F, et al., Thromb Res. 2010 Mar; 125:e110-6). Events included in a range from 0.1 µm to 5 µm were clearly discriminated from background noise. Apoptotic bodies and cell membrane fragments derived from freezing/thawing were stained using SYTOX (Invitrogen-Molecular Probes) and Phalloidin (Invitrogen-Molecular Probes) at RT for 15 and 30 minutes, respectively (Mobarrez F, et al., Thromb Res. 2010 Mar; 125:e110-6). Only SYTOX-/Phalloidin- events were analyzed for the detection of tissue-derived MVs using the appropriate saturating concentrations of the following conjugated monoclonal antibodies: CD235a (BD Biosciences) (Van Beers EJ, et al. Haematologica.2009 Nov;94(11):1513-9); CD61 (BD Biosciences) (Crompot E, et al. PLoS One. 2015 May 15;10(5):e0127209); CD144 (Miltenyi Biotech) (Koga H, et al. J Am Coll Cardiol. 2005 May 17;45(10):1622-30); CD45 (BD Biosciences) (Tähtinen S, et al. Cancer Immunol Res. 2015 May 14); CD14 (BD Horizon) (Griffin JD,et al J. Clin. Invest. 1981; 68: 932-41); CD3ε (BD Biosciences) (Jones et al. 1993; Kothlow et al. 2005); CD73 (BD Biosciences) (Ode A, Eur Cell Mater. 2011 Jul 6;22:26-42)(F. Barry, R. Biochemical and Biophysical Research Communications, vol. 289, no. 2, pp. 519-524, 2001); and CD172a (eBioscience) (Dubois NC, et al. Nat Biotechnol. 2011 Oct 23;29(11):1011-8). MV quality check: Phosphatidylserine (PS) expression was verified in all characterized MV subsets using saturating concentration of annexin V (BD Biosciences).

Monoclonal anti-cardiac troponin T (cTnT) (Innova Biosciences) was used to detect intracellular cTnT in CD172a+ MVs as well as in CD61+ MVs, as an internal negative control, with the Intrasure KiT (BD Biosciences), according to the manufacturer's instructions.

All gated regions were restrictively defined using Fluorescence Minus One (FMO) and/or isotype controls as negative controls. Circulating MV absolute count was obtained with BD Trucount tube (IVD-CE, BD Biosciences), following the manufacturer's protocol. An LSRFortessa analyzer (BD Bioscience) was used for sample acquisition. Data acquisition and analysis were performed with FACSDivav.6.2 (BD Biosciences) and Flow-jo v.9.7 (Tree Star Inc.), respectively. In order to minimize the time-dependent changes in MV count (Lőrincz ÁM, J Extracell Vesicles. 2014 Dec 22;3:25465), the stability of MV quantification was evaluated in five healthy subjects at two time points: baseline (time=0) with fresh isolated MVs, and after one month (time=1M) storage at -80 °C. No differences were observed between the two time-points analysed, suggesting that isolated MVs can be stored for short periods (up to 1M).

### MV sorting.

Circulating MVs were sorted using a FACSAria III cell sorter (Biosciences) equipped with 4 lasers and able to discriminate up to 18 fluorescences. MV sorting was validated using the MegaMix bead (BioCytex). The cell sorter instrument was optimized to sort 0.3 µm, 0.5 µm and 0.9 µm beads with a purity higher than 95%.

### Cardiac-specific microRNA (miRNA) analysis in sorted tissue-derived MVs

Total RNA from sorted cardiac-derived MVs was extracted with TRIzol reagent (Invitrogen, Carlsbad, CA, USA) following the standard protocol. Reverse transcription reactions were performed using the EXIQON miRNA Reverse Transcription Mercury Universal cDNA synthesis kit (Exiqon A/S, Vedbaek, Denmark). Specific primer sequences of cardiac-specific miRNAs (miR-1 [miRBase ID: MIMAT0000416] and miR-133a [miRBase ID: MIMAT0000427]) and miR-21a [miRBase ID: MIMAT0000076], which is not exclusively heart-derived, were obtained (www.exiqon.com/microrna-real-time-pcr-primer-sets) according to literature (Carè et al. Nat Med. 2007 May;13(5):613-8; Catalucci et al. Circ Cardiovasc Genet. 2009 Aug;2(4):402-8). The Freedom Evo150 liquid handling system (Tecan Group, Mannedorf, Switzerland) was used for aliquoting reaction mixtures and samples in all protocol steps. qRT-PCR was performed with a 7900HT Fast Real-Time PCR (Applied Biosystems). miRNA content was evaluated in cardiac-derived MVs as well as in monocyte- and leukocyte-derived MVs, as negative controls, in experimental settings. The miRNA Ct cut off value was considered ≥39.

### Patients enrolment.

All subject has been enrolled, conformed to the ethical guidelines of the 1975 Declaration of Helsinki, as reflected in a priori approval from the Ethical Review Board. Informed consent was obtained from each patient. Enrolment was conducted at Humanitas Clinical and Research Institute, University Hospital of Kiel, Germany, "Federico II" University of Naples, Clinica Mediterranea (Naples, Italy) and AVIS Comunale Milano, (Milan, Italy).

### Aortic Stenosis (AS) cohort.

Patients with Severe symptomatic aortic stenosis (aortic valve area, AVA, <1 cm2; body surface indexed AVA, iAVA, <0.6 cm2/m2)(Svensson LG, Adams DH, Bonow RO, et al. Aortic valve and ascending aorta guidelines for management and quality measures. Ann Thorac Surg 2013;95:S1-66) were recruited at the University Hospital of Kiel, Germany (n=109) and "Federico II" University of Naples (n=10).

AS patients were then undergone percutaneous aortic valve replacement (TAVI). All subjects underwent physical examination, electrocardiogram (ECG), 24-hour ECG Holter monitoring, TTE and Doppler studies, and CMR.

Plasma samples were collected from patients with aortic stenosis (AS) at the time of inclusion (time=0). Clinical follow-up and plasma sampling was performed at 48h, 2 months and 365 days post-surgery.

Negative outcomes during FU period were also recorded.

### Hypertrophic Cardiomyopathy (HCM) cohort.

15 unrelated patients diagnosed with HCM were recruited at the Division of Cardiology, "Federico II" University of Naples, Naples, Italy. The diagnosis of HCM was based on echocardiographic demonstration of a hypertrophied, non-dilated LV (wall thickness >15mm) in the absence of any other cardiac or systemic disorder producing a comparable grade of hypertrophy (Gersh BJ, Maron BJ, Bonow RO, et al. 2011 ACCF/AHA guideline for the diagnosis and treatment of hypertrophic cardiomyopathy: executive summary: a report of the American College of Cardiology Foundation/American Heart Association Task Force on Practice Guidelines. Circulation 2011;124:27861-94). Classification parameters defining HCM status were those established by the American Heart Association guidelines (Gersh BJ, Maron BJ, Bonow RO, et al. 2011 ACCF/AHA guideline for the diagnosis and treatment of hypertrophic cardiomyopathy: executive summary: a report of the American College of Cardiology Foundation/American Heart Association Task Force on Practice Guidelines. Circulation 2011;124:2761-96). None of the patients was in heart failure.

### CAD cohort.

28 patients scheduled between January 7, 2008 and January 31, 2010 to undergo elective DES [drug-eluting stents] implantation at the Clinica Mediterranea (Naples, Italy) were assessed for their suitability for the study. Exclusion criteria were: either non-ST-segment or ST-segment elevation myocardial infarction; cardiogenic shock; allergy/intolerance to aspirin and/or clopidogrel; ongoing serious bleeding or bleeding diathesis; platelet count _75.000/mm3; planned or undelayable noncardiac surgery; previous percutaneous coronary intervention or coronary artery bypass grafting; severe liver disease (e.g., cirrhosis or portal hypertension); and life expectancy <1 year due to other medical conditions. Diabetes mellitus (DM) was diagnosed according to current guidelines (American Diabetes Association. Diagnosis and classification of diabetes mellitus. Diabetes Care 2004;27 Suppl 1:S5-10). Chronic kidney disease (CKD) was defined as an estimated glomerular filtration rate <60 ml/min/ 1.73 m2 (National Kidney Foundation. K/DOQI clinical practice guidelines for chronic kidney disease: evaluation, classification, and stratification. AmJ Kidney Dis 2002;39 Suppl 1:S1-266).

### Control group.

52 healthy subjects were recruited by AVIS (Milan), a multicenter Italian blood donor organization, and Humanitas Research and Clinical Center (Rozzano). Additionally, individuals without cardiovascular disease were obtained from the South Italian Centenarian Study. (Anselmi CV, et al. Rejuvenation Res. 2009 Apr;12(2):95-104). None of the selected healthy subjects had a family history of cardiovascular disease.

### Statistical Methods.

Normality assumption was checked. To evaluate differences between MV-count subsets, Wilcoxon matched pairs test or Mann-Whitney U-test were applied as appropriate. The optimum cutoff value was obtained according to Youden Index (J) and quintile-based method. Clinical Endpoint defined as death was estimated in our cardiac-derived MV by Kaplan-Meier product limit estimator. Comparison of Survival Curves was performed using log-rank Mantel-Cox test. Statistical significance was defined as 2-sided p<0.05 for all tests. The statistical analyses were performed using STATA 11/SE (College Station, Texas) and GraphPad PRISM 5.

### Results

Inventors identified CD172a as surface marker for the identification of cardiac derived MVs in plasma of Aortic Stenosis (AS) patients. In order to further address the cardiac origin of these MVs inventors analysed their internal content by investigating the expression of both cardiac specific Troponin T and micro RNAs. The flow cytometric analysis performed on permeabilized circulating MVs revealed a selective expression of cardiac troponin T in the CD172a+ subpopulation (Fig 4a). Moreover, this result was supported by the real time PCR analysis for Cardiac specific miRNAs performed on sorted CD172a+ MVs. As showed in Figure 4b, the CD172a+ MVs expressed the cardiac specific miR1 and miR133a and the non-cardiac specific miR21 while the CD14+ MVs (internal negative control) expressed only the non-cardiac specific miR21. Altogether these results confirmed the cardiac origin of CD172a+ MVs.

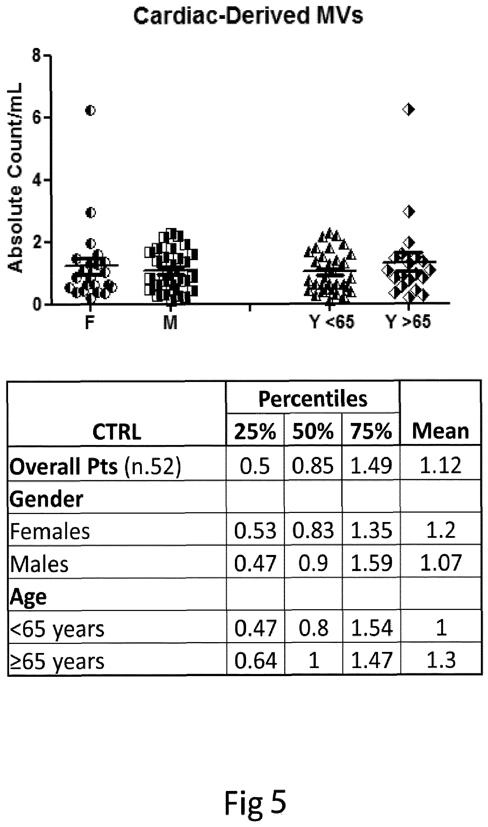

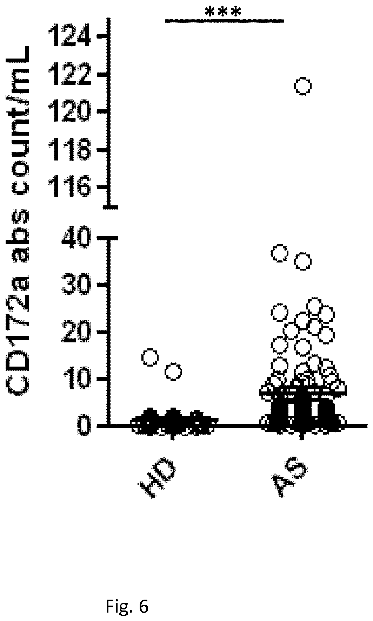

Inventors showed a significant decrease of cardiac MVs (CD172a+) in Aortic Stenosis (AS) patients upon TAVI surgery suggesting the release of these MVs from cardiac cells under stress conditions. In order to compare the absolute count of CD172a+ MVs in AS patients with an enlarged cohort of healthy donors (HD), inventors firstly evaluated the cardiac derived MVs in healthy donors (n=52) stratified for gender and age. In detail, the control group included n.32 individuals aged <65 years (n.8 females and n.24 males; range years: 31-64) and n.20 ≥65 years (n.14 females and n.6 males; range years: 66-98). Noteworthy, the absolute count of circulating CD172a+ MVs showed any significant age- and gender-related link, as reported in Figure 5. Further inventors compared the absolute count of CD172a+ MVs observed in HDs with a cohort of AS patients (n=109) (Figure 6).

Data obtained revealed significant higher level in AS patients' as compared to HDs (***P<0.001) confirming the results obtained by the present inventors.

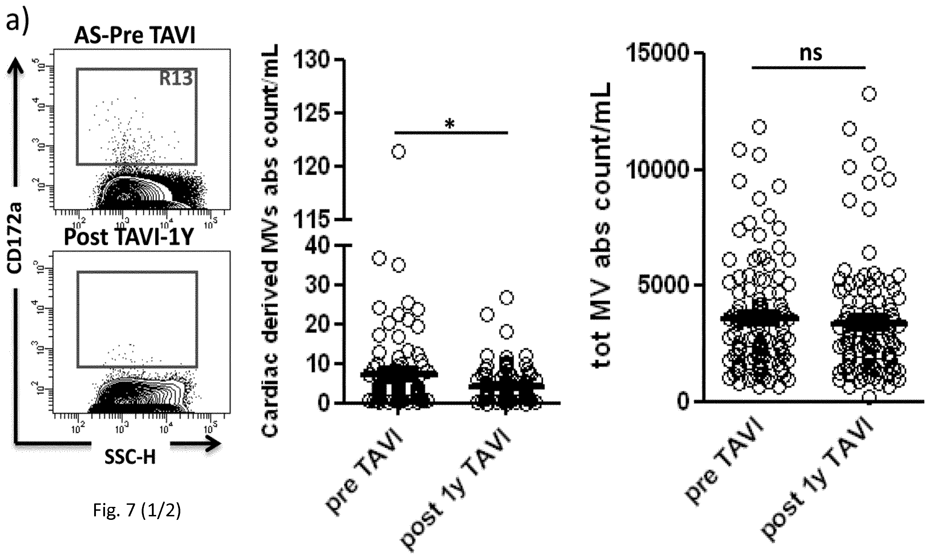

In order to strengthen the data of the cardiac MVs (CD172a+) release in Aortic Stenosis (AS) patients upon 1 year of TAVI surgery (shown in the "Example 1" section), the original cohort was increased for a total of n.109 AS patients. The obtained data showed the selective reduction of the cardiac MVs (*P<0.05) upon TAVI surgery together with no significant fluctuation of the total MVs analysed (Fig. 7a).

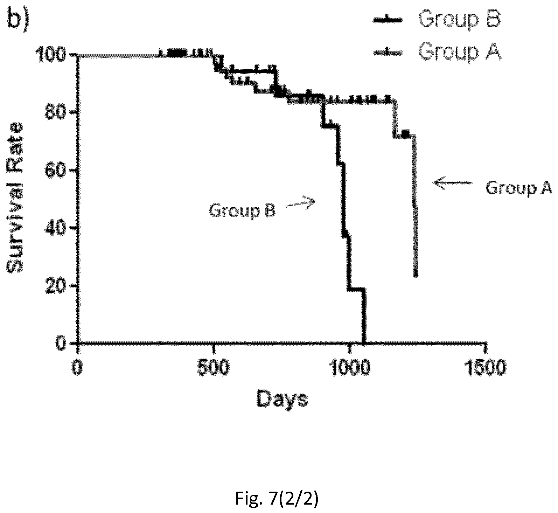

According to defined cut-off (CD172a+ MV absolute count>= 1,79/ml), two AS groups at time of the inclusion was identified: Group A with CD172a+ MV absolute count >=1,79/ml and B with CD172a+ MV absolute count <1,79/ml. Interestingly, the negative outcome rate (i.e. death) was higher in Group B than in Group A (**P<0.01) (Fig. 7b). These findings suggest a potential prognostic relevance of circulating CD172a+ MVs for TAVI treatment (i.e. responder /not responder).

To verify the robustness of the present strategy and the potential role of cardiac-derived MVs as biomarker of myocardial stress, an independentAS cohort was included in the study.

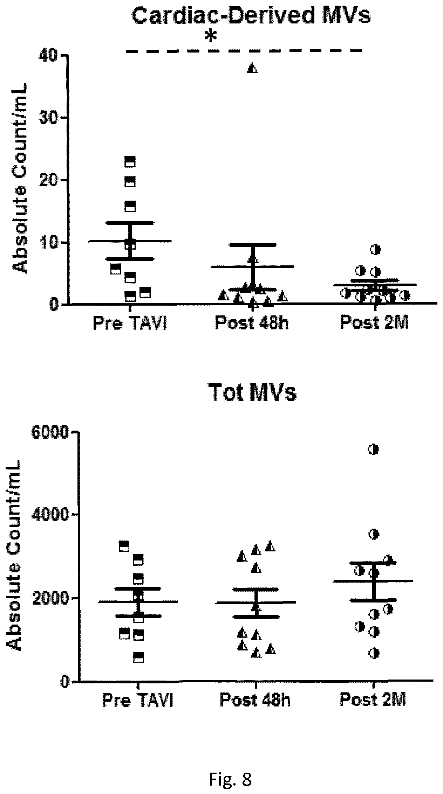

Circulating tissue-derived MVs of n. 10 patients with AS undergone percutaneous aortic valve replacement from "Federico II" University, Naples were evaluated at time of inclusion (pre-TAVI) up to 2-months follow-up, as summarized in Figure 8. The significant reduction of circulating cardiac-derived MV subset was confirmed 2 months after surgery.

Finally, in the effort to better define the pathogenesis of the CD172a+ MV release, two different pathological phenotypes characterized by cardiac suffering: Hypertrophic CardioMyopathy (HCM) (n=15) and Coronary Artery Disease (CAD) (n=28) patients were enrolled. Interestingly, the CD172a+ MVs appeared selectively increased as compared to HDs (*P<0.05 and **P<0.01 respectively) (Fig. 9).

## Claims

1. An ex-vivo or in vitro method for characterizing and/or measuring the amount of subsets of circulating tissue-derived microvesicles (MV) comprising the step of detecting at least the CD172a marker on said microvesicles, in an isolated biological sample obtained from the subject, wherein if the microvesicle is positive for CD172a the microvesicle is characterized as a cardiac derived microvesicle.

2. The ex-vivo or in vitro method according to claim 1 further comprising the step of further detecting at least one marker selected from the group consisting of: CD235a, CD61, CD144, CD14, CD45, CD73, CD3 or any combination thereof.

3. The method according to any one of previous claims wherein the markers to be detected are CD172a, CD235a, CD61, CD144, CD14, CD45 and CD73.

4. The method according to any one of claims 2-3 wherein:
- if the microvesicle is positive for CD235a the microvesicle is characterized as an erythroid derived MV;
- if the microvesicle is positive for CD61 the microvesicle is characterized as a platelet derived MV;
- if the microvesicle is positive for CD144 the microvesicle is characterized as an endothelium-derived MV;
- if the microvesicle is positive for CD14 the microvesicle is characterized as a monocyte-derived MV;
- if the microvesicle is positive for CD45 the microvesicle is characterized as a leukocyte derived MV;
- if the microvesicle is positive for CD73 the microvesicle is characterized as a stromal/adipocyte derived MV.

5. An ex-vivo or in vitro method for diagnosing and/or assessing the risk of developing and/or prognosing and/or for monitoring the progression and/or for monitoring the efficacy of a therapeutic treatment and/or for the screening of a therapeutic treatment of cardiovascular diseases (CVD), in a subject comprising the steps of:
a) characterizing and/or measuring the amount of subsets of circulating tissue-derived microvesicles according to the method of any of claims 1-4;
b) comparing with respect to a proper control and/or reference.

6. The method according to claim 5, wherein an amount of cardiac-derived MV, in the isolated biological sample obtained from the subject, higher than the control amount indicates that the subject is either affected by or is at increased risk for developing cardiovascular diseases (CVD).

7. The method according to claim 5, wherein an amount of cardiac-derived MV, in the isolated biological sample obtained from the subject, lower than the control amount indicates that the subject is going toward an amelioration of the CVD, preferably wherein the subject undergone a valve replacement, preferably a transcatheter aortic valve implantation (TAVI).

8. The method according to any one of claims 5-7 wherein the cardiovascular disease (CVD) is selected from the group consisting of: heart failure, preferably primary or secondary, aortic stenosis (AS), valvular disease, cardiomyopathy, acute coronary artery disease (CAD), atherosclerosis, myocardial ischemia, infarction, arrhythmias, hypertrophic cardiomyopathy (HCM) and drug-dependent cardiotoxicity connected to different pathologies (e.g. cancer, HIV-HAART therapies).

9. The method according to any one of any one of claims 5-8 wherein the measured or characterized subset of circulating tissue-derived microvesicles is the cardiac-derived MV subset, preferably wherein the cardiac-derived MV subset is **characterized by** the presence of the marker CD172a, more preferably wherein the cardiac derived microvesicles are negative for the following markers: CD235a, CD61, CD144, CD14, CD45, CD73.

10. The method according to any one of claims 5-9 wherein the isolated biological sample is plasma, blood, serum, tissue obtained by surgical resection, tissue obtained by biopsy, cells culture, cell supernatants, cell lysates, tissue samples, organs, bone marrow.

11. The method according to any one of the previous claims wherein the marker is detected by means of a specific ligand, preferably wherein the ligand is an antibody, or functional fragment thereof.

12. The method according to claim 11, wherein the marker is detected by magnetic beads coated with antibody capture and/or customized dried antibody cocktails and/or columns with sized filter cartridges and/or combined with specific antibody filter (SAF).

13. The method according to any one of previous claims wherein the subsets of circulating tissue-derived microvesicles are characterized and/or their amount measured by means of multicolor flow cytometry technology (FACS), immunogold electron microscopy, immunofluorescence, ELISA, immunoprecipitation, reverse colorimetric immunoassay (RCIA), radioimmune assay (RIA) Electrochemiluminescence, surface plasmon resonance (SPR)-based approach, nanoliter microfluidics (immunoassays) or spectometry.

14. Use of a ligand specific for CD172a for the detection and/or quantification of cardiac-derived MVs in an isolated biological sample, preferably wherein the ligand is an anti-CD 172a antibody, or functional fragments thereof.

## Patentansprüche

1. Ex-vivo- oder in-vitro-Verfahren zur Charakterisierung und/oder Messung der Menge an Untergruppen von zirkulierenden, von Gewebe abgeleiteten Mikrovesikeln (MV), umfassend den Schritt des Detektierens wenigstens des CD172a-Markers an den Mikrovesikeln, in einer isolierten biologischen Probe, die von dem Individuum gewonnen wurde, wobei, wenn das Mikrovesikel positiv für CD172a ist, das Mikrovesikel als ein vom Herzen abgeleitetes Mikrovesikel charakterisiert wird.

2. Ex-vivo- oder in-vitro-Verfahren nach Anspruch 1, ferner umfassend den Schritt des weiteren Detektierens wenigstens eines Markers, ausgewählt aus der Gruppe bestehend aus: CD235a, CD61, CD144, CD14, CD45, CD73, CD3 oder jeder Kombination davon.

3. Verfahren nach einem der vorhergehenden Ansprüche, wobei die zu detektierenden Marker CD172a, CD235a, CD61, CD144, CD14, CD45 und CD73 sind.

4. Verfahren nach einem der Ansprüche 2 bis 3, wobei:
- wenn das Mikrovesikel positiv für CD235a ist, das Mikrovesikel als ein von Erythroiden abgeleitetes MV charakterisiert wird;
- wenn das Mikrovesikel positiv für CD61 ist, das Mikrovesikel als ein von Thrombozyten abgeleitetes MV charakterisiert wird;
- wenn das Mikrovesikel positiv für CD144 ist, das Mikrovesikel als ein von Endothel abgeleitetes MV charakterisiert wird;
- wenn das Mikrovesikel positiv für CD14 ist, das Mikrovesikel als ein von Monozyten abgeleitetes MV charakterisiert wird;
- wenn das Mikrovesikel positiv für CD45 ist, das Mikrovesikel als ein von Leukozyten abgeleitetes MV charakterisiert wird;
- wenn das Mikrovesikel positiv für CD73 ist, das Mikrovesikel als ein von Stromazellen/Adipozyten abgeleitetes MV charakterisiert wird.

5. Ex-vivo- oder in-vitro-Verfahren zur Diagnostizierung und/oder Beurteilung des Risikos einer Entstehung und/oder zur Voraussage und/oder zur Überwachung des Fortschritts und/oder zur Überwachung der Wirksamkeit einer therapeutischen Behandlung und/oder zum Screening einer therapeutischen Behandlung von kardiovaskulären Erkrankungen (CVD) in einem Individuum, umfassend die folgenden Schritte:
a) Charakterisieren und/oder Messen der Menge an Untergruppen von zirkulierenden, von Gewebe abgeleiteten Mikrovesikeln nach dem Verfahren von einem der Ansprüche 1 bis 4;
b) Vergleichen in Bezug auf eine geeignete Kontrolle und/oder Referenz.

6. Verfahren nach Anspruch 5, wobei eine Menge an vom Herzen abgeleiteten MV in der von dem Individuum gewonnenen, isolierten biologischen Probe, die höher als die Kontrollmenge ist, anzeigt, dass das Individuum entweder an kardiovaskulären Erkrankungen (CVD) leidet oder ein erhöhtes Risiko aufweist, solche zu entwickeln.

7. Verfahren nach Anspruch 5, wobei eine Menge an vom Herzen abgeleiteten MV in der von dem Individuum gewonnenen, isolierten biologischen Probe, die niedriger ist als die Kontrollmenge, anzeigt, dass das Individuum auf eine Verbesserung der CVD zugeht, wobei sich das Individuum vorzugsweise einem Klappenersatz, vorzugsweise einer Transkatheter-Aortenklappenimplantation (TAVI) unterzogen hat.

8. Verfahren nach einem der Ansprüche 5 bis 7, wobei die kardiovaskuläre Erkrankung (CVD) aus der Gruppe bestehend aus: Herzinsuffizienz, vorzugsweise primärer oder sekundärer Aortenstenose (AS), Klappenerkrankung, Kardiomyopathie, akuter Koronararterienerkrankung (CAD), Atherosklerose, Myokardischämie, Infarkt, Rhythmusstörungen, hypertropher Kardiomyopathie (HCM) und arzneimittelbedingter Kardiotoxizität, die mit verschiedenen Erkrankungen (z.B. Krebs, HIV-HAART-Therapien) verbunden ist, ausgewählt wird.

9. Verfahren nach einem der Ansprüche 5 bis 8, wobei die gemessene oder charakterisierte Untergruppe von zirkulierenden, von Gewebe abgeleiteten Mikrovesikeln die vom Herzen abgeleitete MV-Untergruppe ist, wobei die vom Herzen abgeleitete MV-Untergruppe vorzugsweise durch das Vorhandensein des Markers CD172a gekennzeichnet ist, wobei die vom Herzen abgeleiteten Mikrovesikel stärker bevorzugt negativ für die folgenden Marker sind: CD235a, CD61, CD144, CD14, CD45, CD73.

10. Verfahren nach einem der Ansprüche 5 bis 9, wobei es sich bei der isolierten biologischen Probe um Plasma, Blut, Serum, durch chirurgische Resektion gewonnenes Gewebe, durch Biopsie gewonnenes Gewebe, eine Zellkultur, Zellüberstände, Zelllysate, Gewebsproben, Organe, Knochenmark handelt.

11. Verfahren nach einem der vorhergehenden Ansprüche, wobei der Marker mithilfe eines spezifischen Liganden detektiert wird, wobei der Ligand vorzugsweise ein Antikörper oder ein funktionelles Fragment davon ist.

12. Verfahren nach Anspruch 11, wobei der Marker detektiert wird durch Magnetkügelchen, die mit einem Antikörpereinfang beschichtet sind, und/oder individualisierte getrocknete Antikörpercocktails und/oder Säulen mit angepassten Filterkartuschen und/oder kombiniert mit einem spezifischen Antikörperfilter (SAF).

13. Verfahren nach einem der vorhergehenden Ansprüche, wobei die Untergruppen von zirkulierenden, von Gewebe abgeleiteten Mikrovesikeln wie folgt charakterisiert werden und/oder ihre Menge wie folgt gemessen wird: durch Mehrfarben-Durchflusszytometrietechnologie (FACS), Immungold-Elektronenmikroskopie, Immunfluoreszenz, ELISA, Immunpräzipitation, reversen kolorimetrischen Immunassay (RCIA), Radioimmunassay (RIA), Elektrochemilumineszenz, einen Oberflächenplasmonresonanz-(SPR)-basierten Ansatz, Nanoliter-Mikrofluidik (Immunoassays) oder Spektrometrie.

14. Verwendung eines für CD172a spezifischen Liganden zur Detektion und/oder Quantifizierung von vom Herzen abgeleiteten MVs in einer isolierten biologischen Probe, wobei es sich bei dem Liganden vorzugsweise um einen Anti-CD172a-Antikörper oder funktionelle Fragmente davon handelt.

## Revendications

1. Procédé ex vivo ou in vitro de caractérisation et/ou de mesure de la quantité de sous-ensembles de microvésicules (MV) circulantes d'origine tissulaire comprenant l'étape de détection d'au moins un marqueur CD172a sur lesdites microvésicules, dans un échantillon biologique isolé obtenu du sujet, dans lequel si la microvésicule est positive pour CD172a la microvésicule est caractérisée comme une microvésicule d'origine cardiaque.

2. Procédé ex vivo ou in vitro selon la revendication 1 comprenant en outre l'étape de détection supplémentaire d'au moins un marqueur sélectionné dans le groupe consistant en : CD235a, CD61, CD144, CD14, CD45, CD73, CD3 ou toute combinaison de ceux-ci.

3. Procédé selon l'une quelconque des revendications précédentes dans lequel les marqueurs à détecter sont CD172a, CD235a, CD61, CD144, CD14, CD45 et CD73.

4. Procédé selon l'une quelconque des revendications 2 et 3 dans lequel :
- si la microvésicule est positive pour CD235a la microvésicule est caractérisée comme une MV d'origine érythroïde ;
- si la microvésicule est positive pour CD61 la microvésicule est caractérisée comme une MV d'origine plaquettaire ;
- si la microvésicule est positive pour CD144 la microvésicule est caractérisée comme une MV d'origine endothéliale ;
- si la microvésicule est positive pour CD14 la microvésicule est caractérisée comme une MV d'origine monocytaire ;
- si la microvésicule est positive pour CD45 la microvésicule est caractérisée comme une MV d'origine leucocytaire ;
- si la microvésicule est positive pour CD73 la microvésicule est caractérisée comme une MV d'origine adipocytaire/stromale.

5. Procédé ex vivo ou in vitro de diagnostic et/ou d'évaluation du risque de développement et/ou de pronostic et/ou de surveillance de la progression et/ou de surveillance de l'efficacité d'un traitement thérapeutique et/ou de sélection d'un traitement thérapeutique de maladies cardiovasculaires (MCV), chez un sujet comprenant les étapes suivantes :
a) caractérisation et/ou mesure de la quantité de sous-ensembles de microvésicules circulantes d'origine tissulaire conformément au procédé selon l'une quelconque des revendications 1 à 4 ;
b) comparaison par rapport à un témoin et/ou une référence corrects.

6. Procédé selon la revendication 5, dans lequel une quantité de MV d'origine cardiaque, dans l'échantillon biologique isolé obtenu du sujet, supérieure à la quantité témoin indique que le sujet soit est affecté par une, soit présente un risque accru de développement d'une, maladie cardiovasculaire (MCV).

7. Procédé selon la revendication 5, dans lequel une quantité de MV d'origine cardiaque, dans l'échantillon biologique isolé obtenu du sujet, inférieure à la quantité témoin indique que le sujet va vers une amélioration de la MCV, de préférence dans lequel le sujet subit un remplacement de valvule, de préférence une implantation de valvule aortique transcathéter (TAVI).

8. Procédé selon l'une quelconque des revendications 5 à 7, dans lequel la maladie cardiovasculaire (MCV) est sélectionnée dans le groupe consistant en : une insuffisance cardiaque, de préférence primaire ou secondaire, une sténose aortique (SA), une maladie valvulaire, une cardiomyopathie, une maladie coronarienne aiguë (CAD), l'athérosclérose, une ischémie du myocarde, un infarctus, des arythmies, une cardiomyopathie hypertrophique (HCM) et une cardiotoxicité dépendante des médicaments associée à différentes pathologies (par exemple le cancer, une thérapie HAART contre le VIH).

9. Procédé selon l'une quelconque des revendications 5 à 8 dans lequel le sous-ensemble mesuré ou caractérisé de microvésicules circulantes d'origine tissulaire est le sous-ensemble de MV d'origine cardiaque, de préférence dans lequel le sous-ensemble de MV d'origine cardiaque est **caractérisé par** la présence du marqueur CD172a, de manière davantage préférée dans lequel les microvésicules d'origine cardiaques sont négatives pour les marqueurs suivants : CD235a, CD61, CD144, CD14, CD45, CD73.

10. Procédé selon l'une quelconque des revendications 5 à 9 dans lequel l'échantillon biologique isolé est le plasma, le sang, le sérum, un tissu obtenu par résection chirurgicale, un tissu obtenu par biopsie, une culture cellulaire, des surnageants cellulaires, des lysats cellulaires, des échantillons de tissu, des organes, de la moelle osseuse.

11. Procédé selon l'une quelconque des revendications précédentes dans lequel le marqueur est détecté au moyen d'un ligand spécifique, de préférence dans lequel le ligand est un anticorps, ou un fragment fonctionnel de celui-ci.

12. Procédé selon la revendication 11, dans lequel le marqueur est détecté par des billes magnétiques revêtues de cocktails de capture d'anticorps et/ou d'anticorps secs personnalisés et/ou des colonnes avec des cartouches filtrantes calibrées et/ou combinées à des filtres à anticorps spécifiques (SAF).

13. Procédé selon l'une quelconque des revendications précédentes dans lequel les sous-ensembles de microvésicules circulantes d'origine tissulaire sont caractérisés et/ou leur quantité mesurée à l'aide de la technologie de cytométrie en flux multicolore (FACS), de la microscopie électronique immunogold, de l'immunofluorescence, d'un ELISA, de l'immunoprécipitation, d'un dosage immuno-colorimétrique inverse (RCIA), d'un dosage radio-immunologique (RIA), de l'électrochimioluminescence, d'une approche basée sur la résonance plasmonique de surface (SPR), de la microfluidique (des dosages immunologiques microfluidiques) à l'échelle du nanolitre ou de la spectrométrie.

14. Utilisation d'un ligand spécifique de CD172a pour la détection et/ou la quantification de MV d'origine cardiaque dans un échantillon biologique isolé, de préférence dans lequel le ligand est un anticorps anti-CD172a, ou des fragments fonctionnels de celui-ci.
